# EUROPEAN PATENT SPECIFICATION

(11) **EP 2 029 287 B1**
(45) Date of publication and mention of the grant of the patent: **13.12.2017**
(21) Application number: 07729686.1
(22) Date of filing: 30.05.2007
(51) Int. Cl.: B05B 11/00

(54) **FLUID DISPENSER**
FLUIDSPENDER
DISTRIBUTEUR DE FLUIDE

(30) Priority: 30.05.2006 GB 0610666
(43) Date of publication of application: 04.03.2009
(73) Proprietor: Glaxo Group Limited, Brentford, Middlesex TW8 9GS (GB)
(72) Inventor: LINTERN, Richard, David, Huntingdon Cambridgeshire PE28 3AS (GB); PEARSON, Allen, John, Huntingdon Cambridgeshire PE28 3AS (GB); RAND, Paul, Kenneth, Ware Hertfordshire SG12 0DP (GB)
(74) Representative: Rice, Jason Neale
(86) International application number: PCT/EP2007/055273
(87) International publication number: WO 2007/138084

(56) References cited:
- EP-A- 0 688 608
- WO-A-2005/075103
- FR-A- 2 708 314
- FR-A- 2 852 934
- FR-A- 2 862 498
- FR-A1- 2 674 747
- FR-A1- 2 815 611
- FR-A1- 2 862 009
- GB-A- 2 002 847
- US-A- 5 024 355

## Description

### Field of the Invention

The present invention relates to a fluid dispenser, for example for a nasal spray, and is particularly, but not exclusively, concerned with a fluid dispenser for drug administration.

### Background of the Invention

Prior art fluid dispensers, e.g. for dispensing fluids into a nasal cavity, are known from US-A-2005/0236434 and WO-A-2005/075103. These dispensers comprise a fluid reservoir, an outlet and a pump for pumping fluid from the reservoir through the outlet. The outlet is provided in a nozzle, which nozzle may be shaped and sized for positioning in a nostril. As the dispensers are for dispensing a metered volume of the fluid, they further comprise a metering chamber which is selectively placed in fluid communication with the reservoir, through at least one metering chamber inlet, and the outlet. The pump reciprocates to move the metering chamber between an expanded state, in which the metering chamber has a first volume greater than the metered volume, and a contracted state. The dispensers further comprise a one-way valve between the metering chamber and the outlet which is biased to a 'valve-closed' position. When the metering chamber moves from its contracted state to its expanded state, the metering chamber and reservoir are placed in fluid communication through the at least one inlet and fluid is drawn from the reservoir into the metering chamber to fill the metering chamber with an excess volume of fluid. When the metering chamber moves from the expanded state towards the contracted state, there is an initial bleed phase in which the surplus volume of fluid in the metering chamber is pumped back into the reservoir through the at least one inlet to leave a metered volume of fluid in the metering chamber. In a final dispensing phase of movement of the metering chamber back to its contracted state, the metered volume of fluid in the metering chamber is pumped towards the one-way valve whereby the increasing pressure produced in the fluid causes the one-way valve to temporarily open to enable the metered volume to be pumped from the outlet.

An aim of the present invention is to provide a novel fluid dispenser, optionally incorporating the pumping principle disclosed in US-A-2005/0236434 and WO-A-2005/075103.

A fluid dispenser according to the pre-characterising part of claim 1 appended hereto is known from FR-A-2815611.

### Summary of the Invention

According to the invention there is provided a fluid dispenser according to claim 1 appended hereto.

Some of the preferred features of the invention are set out in the other claims appended hereto.

Preferably the fluid dispenser comprises a nozzle for inserting into a nostril of a user. Preferably the fluid outlet is formed in the nozzle. Most preferably it is formed at an outer end of the nozzle. The nozzle may be formed as a one-piece part.

Preferably the fluid dispenser comprises a supply of fluid, for example in the form of a fluid reservoir. The supply may be contained in a receptacle. The receptacle may be vented, or may be non-vented, e.g. of a variable internal volume, for instance contractible in response to fluid being removed therefrom, for example by having a moveable plunger.

Preferably the fluid dispenser is adapted to be repeatedly operated to dispense on each operation a dose of the fluid though the fluid outlet. To this end, the supply is preferably a supply containing multiple doses of the fluid.

The invention may also comprise any of the additional features of the embodiments described with reference to the accompanying Figures.

The present invention will be understood from the embodiments which will now be described with reference to the accompanying Figures of drawings.

### Brief Description of the Figures of Drawings

Figs. 1 to 4 schematically illustrate a sequence of operational steps carried out on a fluid dispenser embodying various aspects;
Figures 5 to 8 illustrate an alternative seal arrangement for the fluid dispenser;
Figures 9 to 14 illustrate how to assemble an embodiment;
Figures 15 and 16 show another alternative seal arrangement;
Figures 17 to 20 illustrate a sequence of operational steps carried out on another embodiment featuring another alternative seal arrangement;
Figure 21 shows yet another alternative seal arrangement;
Figures 22A to 22C are perspective side views of a fluid dispenser in accordance with the present invention, where Figure 22A shows the fluid dispenser in a fully extended (open) position and Figures 22B and 22C respectively show the fluid dispenser in its rest and fired positions;
Figures 23A to 23C illustrate the assembly of the fluid dispenser of Figures 22A-C;
Figures 24A to 24C are cross-sectional side views of the fluid dispenser of Figures 22A-C in its fully extended, rest and fired positions;
Figure 25 is an enlarged cross-sectional view of the nozzle area of the fluid dispenser of Figures 22 to 24 showing an alternative tip seal arrangement;
Figures 26A and 26B are respectively side views and cross-sectional side views of a piston member of the fluid dispenser of Figures 22 to 25;
Figures 27A and 27B are respectively perspective and cross-sectional side views of a rear sealing element of the fluid dispenser of Figures 22 to 25 which mounts on the piston member of Figures 26A-B;
Figures 28A and 28B are respectively perspective and cross-sectional side views of a forward sealing element of the fluid dispenser of Figures 22 to 25 which slidably mounts on the piston member of Figures 26A-B to form a one-way valve;
Figures 29A and 29B are respectively perspective and cross-sectional side views of a main housing of the fluid dispenser of Figures 22 to 25 which slidingly receives the piston member of Figures 26A-B;
Figures 30A and 30B are respectively perspective and cross-sectional side views of a stopper portion of the fluid dispenser of Figures 22 to 25 which mounts on a fluid supply and to which mounts the piston member of Figures 26A-B;
Figures 31A and 31B are respectively perspective and cross-sectional side views of a nozzle of the fluid dispenser of Figures 22 to 25 which slidingly mounts on the stopper portion of Figures 30A-B;
Figure 32 is a perspective rear view of the nozzle of Figures 31A and 31B showing a swirl chamber formed in the end face thereof;
Figures 33A and 33B are respectively perspective and cross-sectional side views of a carrier member of the fluid dispenser of Figures 22 to 25 which slidingly mounts on the nozzle of Figures 31A-B and 32;
Figures 34A and 34B are perspective views of a valve element of a valve mechanism of the fluid dispenser of Figures 22 to 25 which mounts in the main housing of Figures 29A-B;
Figures 35A and 35B are respectively perspective and cross-sectional side views of a nozzle insert of the fluid dispenser of Figures 22 to 25 which inserts in the nozzle of Figures 31A-B and 32;
Figures 36A and 36B are respectively perspective and cross-sectional side views of a cap of the fluid dispenser of Figures 22 to 25 which mounts on the main housing of Figures 29A-B;
Figures 37A to 37J are cross-sectional side views of a modified version of the fluid dispenser of Figures 22 to 36 showing the sequential advancement of liquid therewithin during priming of the dispenser;
Figure 38 corresponds to Figure 32 showing an modification to the swirl chamber;
Figure 39 corresponds to Figure 25, but shows an alternative tip seal arrangement for the fluid dispenser of Figures 22 to 36;
Figure 40 corresponds to Figure 25, but shows a further alternative tip seal arrangement;
Figures 41A and 41B are respectively perspective and cross-sectional side views of the nozzle insert in Figure 40;
Figure 42 corresponds to Figure 25, but shows an alternative sealing arrangement for the fluid dispenser of Figures 22 to 36;
Figures 43A and 43B are respectively perspective and cross-sectional side views of the sealing pin in Figure 42;
Figures 44A and 44B are respectively perspective and cross-sectional side views of the backing plate in Figure 42;
Figures 45A and 45B are respectively perspective and cross-sectional side views of the nozzle insert in Figure 42; and
Figures 46A and 46B are respectively perspective and cross-sectional side views of the forward sealing element in Figure 42.

### Detailed Description of the Figures of Drawings

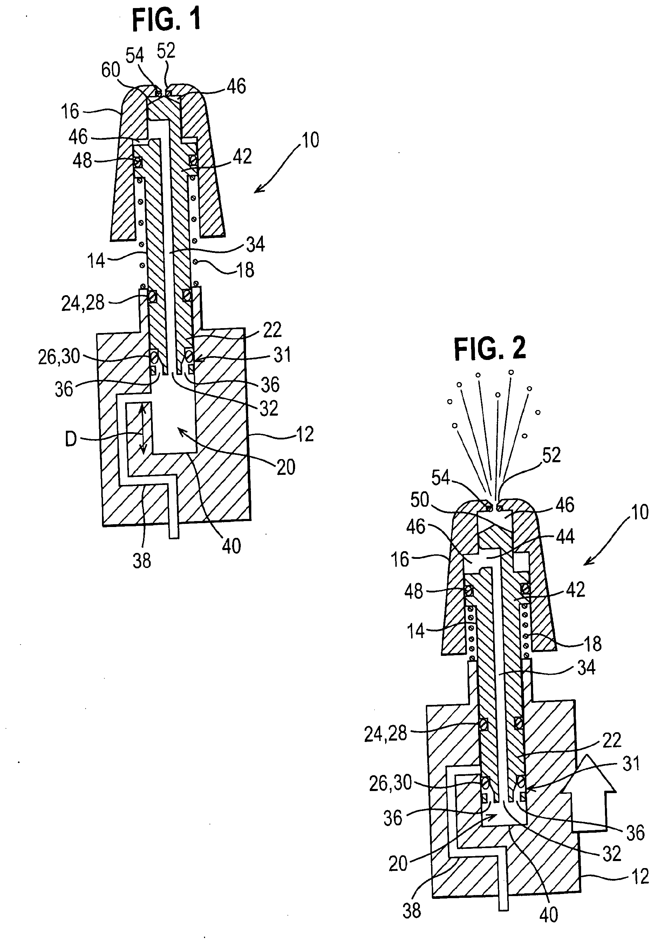

Referring first of all to Figures 1 to 4, there is shown a schematic representation of the sequence of operation of a fluid dispenser 10, in this instance for dispensing a liquid containing a medicament, for example suspended or dissolved in the liquid. The underlying principle of operation of the fluid dispenser 10 is as described in US-A-2005/0236434 and WO-A-2005/075103 *supra.*

The fluid dispenser 10 comprises a main housing 12, a piston member 14, a nozzle 16 and a spring 18. The spring 18 is for biasing the nozzle 16 away from, and piston member 14 out of, the main housing 12. The skilled reader will appreciate that the nozzle 16 could form an internal component of the fluid dispenser 10, e.g. housed within a dispenser casing (not shown).

The main housing 12 has an internal cavity that defines a dosing chamber 20. That dosing chamber 20, in this preferred embodiment, has a cylindrical cross-section. The dosing chamber 20 in this particular embodiment forms a metering chamber which meters a volume of the fluid for dispensement from the dispenser 10, as in US-A-2005/0236434 and WO-A-2005/075103 *supra.*

A first end 22 of the piston member 14 also has a generally cylindrical cross-section. The diameter of that first end 22, however, is smaller than the diameter of the dosing chamber 20. As a result, that first end 22 of the piston member 14 will freely slide within the dosing chamber 20. However, to stop that, that first end 22 of the piston member 14 is also provided with two annular grooves 24, 26 around its circumference, with each annular groove 24, 26 having an O-ring 28, 30 positioned in it. Those O-rings 28, 30 extend above the surface of the first end 22 of the piston member 14 so as to seal the gap between the piston member 14 and the wall of the dosing chamber 20. As a result, the first end 22 of the piston member 14 can act as a piston within the dosing chamber 20. As a piston, it will impose a pumping force onto fluid within the dosing chamber 20 as the piston member 14 moves within the dosing chamber 20.

The end wall of the first end 22 (i.e. the bottom end) of the piston member 14 faces into the dosing chamber 20. A hole 32 is provided in the middle of that end wall. That hole is an entrance hole for a fluid conduit 34 that extends along almost the full length of the piston member 14. That fluid conduit 34 is for feeding fluid from the dosing chamber 20 into a fluid dispensement chamber 46 in the nozzle 16 upon actuation of the fluid dispenser 10 for dispensement of fluid out of the nozzle 16.

Spaced around the entrance hole 32 in the bottom end 22 of the piston member 14 is a further annular groove. This further annular groove is provided as a circular groove 36 in that bottom wall, rather than extending around the side wall of the piston member 14. This circular groove 36 is in fluid communication with the second annular groove 26, i.e. the groove that is otherwise closest to the bottom end 22 of the piston member 14. The fluid communication between these two annular grooves 26, 36 may be achieved with intermittent slots or holes between the two grooves 26, 36. A single slot or hole would function however.

The combination of the circular groove 36, the second annular groove 26 and the second O-ring 30 provides a non-return valve 31 at the first end 22 of the piston member 14, as will now be described.

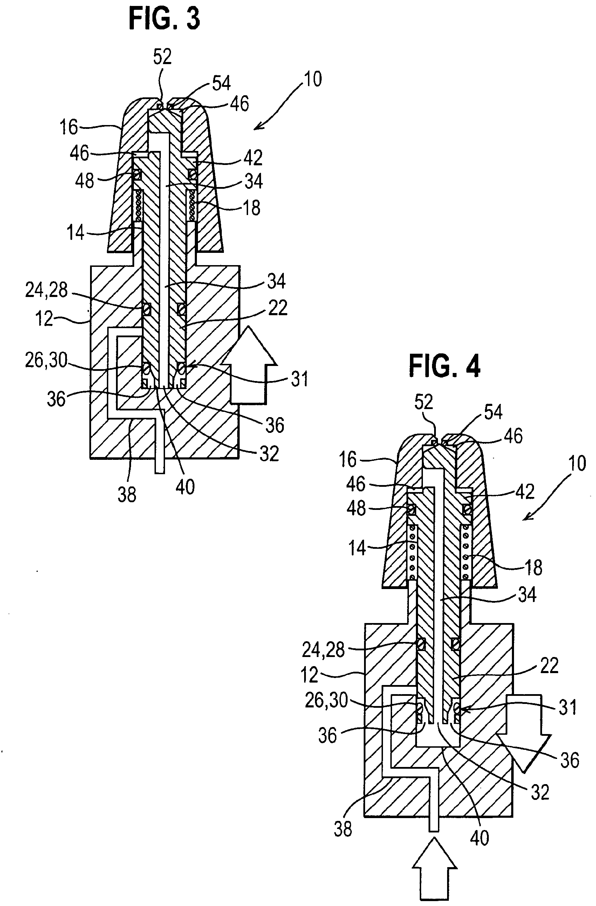

The width of the annular groove 26 is greater than the width dimension taken by its O-ring 30 when that O-ring 30 is compressed against the side wall of the dosing chamber 20. As a result, that O-ring 30 can move within the annular groove 26 between two positions - a forward, sealing position (farther away from the bottom end 22) and a backward, non-sealing position (closer to the bottom end 22). Figs 3 and 4, respectively, show these two positions. As can be seen, the movement is in a direction that is generally parallel to the axis of the piston member 14.

The second annular groove 26 also has a ramped base, whereby it has a varying depth. That varying depth allows the groove 26 to define two annular regions, the first annular region being for receiving the O-ring 30 at its forward, sealing position, and being spaced farthest from the circular groove 36, and the second annular region being for receiving the O-ring 30 at its backward, non-sealing position, and being spaced closer to the circular groove 36 than the first annular region.

The ramped base is arranged such that the first annular region is less deep than the second annular region. The depth transition may be created by a straight ramp, or it may be created by either a curved ramp (usually a convex curve) or a ramp with one or more landings, or flat (non-depth-varying) regions. In the embodiment illustrated in Figure 1, there is a convexly curved base.

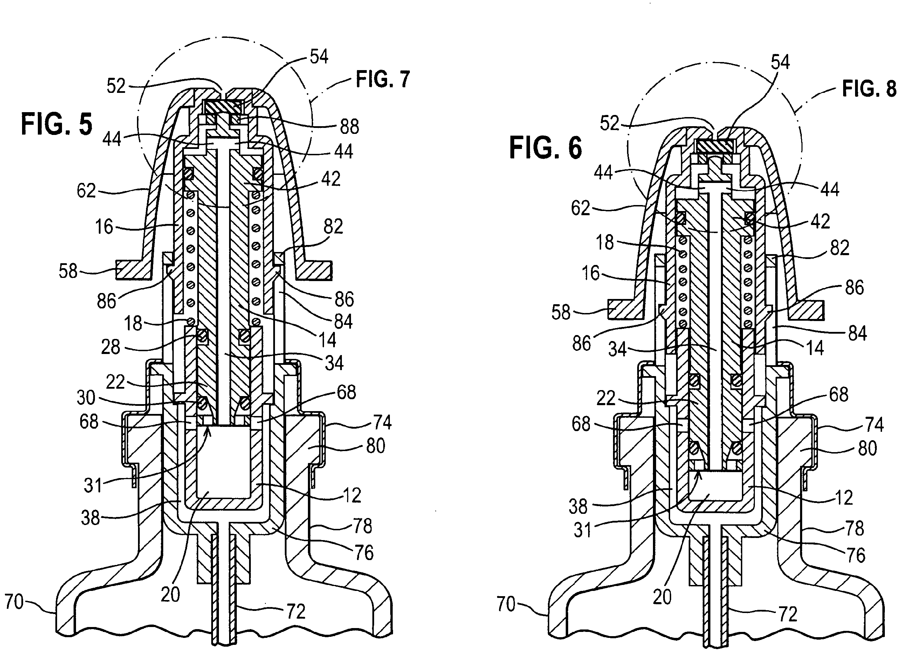

The non-return valve 31 functions as follows:
a) When the O-ring 30 is positioned in the first annular region, the O-ring 30 seals or closes the non-return valve 31 by being pressed both against the side wall of the dosing chamber 20 and against the base of the first annular region. This occurs because the first annular region has a diameter that is equal to, or more typically, greater than the inside diameter of the O-ring 30, and also because the outside diameter of the O-ring 30 is equal to, or more typically, slightly larger than, the diameter of the dosing chamber 20. When sealed like this, fluid will not pass around the O-ring 30 and thus not through the valve 31.
b) When the O-ring 30 has moved into the second annular region, due to the piston member 14 moving relative to the dosing chamber 20, the O-ring 30 becomes loose within the annular groove 26. That is because the ramp, in effect, retracts the base of the groove 26 away from the O-ring's innermost surface, whereupon it will no longer be pressed against the base of the annular groove 26 as well as the side wall of the dosing chamber 20 - that second annular region, as explained above, is deeper than the first annular region and has a diameter that is smaller than the inside diameter of the O-ring 30. The non-return valve 31 is then open, as fluid can pass around the O-ring 30, whereupon fluid can pass through the valve 31 into the dosing chamber 20 from a source of fluid (e.g. a bottle 70 - see Figs 5, 6 and 14) via a second fluid conduit 38. As will be seen from Figure 4, for example, the inherent bias in the O-ring 30 maintains the outer surface of the O-ring 30 in contact with the sidewall of the dosing chamber 20, thereby creating a gap between the inner diametric surface of the O-ring 30 and the base of the second annular region. Accordingly, the fluid flows through this gap into the dosing chamber 20.

When the piston member 14 moves downward relative to the dosing chamber 20, the O-ring 30 is disposed in its forward, sealing position, as shown in Figures 2 and 3. Conversely, when the piston member 14 moves upwardly relative to the dosing chamber 20, the O-ring 30 is disposed in its backward, non-sealing position, as shown in Figures 1 and 4. As will be understood by the skilled reader, the O-ring 30 is moved between its forward and backward positions in the groove 26 simply by the movement of the piston member 14 relative to the dosing chamber 20.

The second fluid conduit 38 introduces fluid into the dosing chamber 20 through the side wall of the dosing chamber 20. That entrance point is located a fixed distance D from the bottom wall 40 of the dosing chamber 20 (see Fig. 1). That distance D sets the metered volume of fluid to be dispensed by the dispenser upon each full actuation i.e. the metered volume is the volume of the dosing chamber below that point, for example 50µl (microlitres). It should be noted, however, that by varying that distance D, different dispensement volumes can be provided. That variation might be achieved by moulding the entrance point in a different location, e.g. in the factory, or by providing a moveable/variable position for the entrance point.

As shown in Figure 1 (the fully extended, rest position), the piston member's bottom end 22 is located above the entrance point for the second fluid conduit 38. The dosing chamber is overfilled at that time - the dosing chamber is full and has, in this rest position, a volume greater than that which is to be dosed from the dispenser 10. However, this overfilling will not result in an overdose to a user. As will be understood from Figures 2 and 3, this is because upon compressing the piston member 14 back down into the dosing chamber 20, the excess volume of fluid (i.e. the fluid above the entrance point) will be forced/pumped back out of the dosing chamber 20 through the entrance point and down through second fluid conduit 38 and back into the bottle, until the O-ring 30 (which is in its forward, sealing position) closes the entrance point. Thereafter the fluid volume in the dosing chamber 20 is fixed by the non-return valve 31, i.e. the metered volume is defined.

The above described overfilling upon each actuation cycle serves a useful function. It ensures that a complete and accurate metered dosage is provided upon each actuation.

Although only one entrance point to the dosage chamber 20 is shown, more than one entrance point may be provided, e.g. as shown in WO-A-2005/075103 supra. This reduces flow resistance between the dosing chamber 20 and the source of fluid.

In Figure 4, the downward arrow shows that the main housing 12 is being moved away relative to the nozzle 16 and the piston 14. This may be achieved by moving the housing 12 downwardly whilst the piston 14 and nozzle 16 are static or by simultaneously moving the piston 14 and nozzle 16 upwardly with the housing 12 being static, or by simultaneously moving the housing 12 downwards and the piston 14 and nozzle 16 upwards. Irrespective, the upward arrow indicates the resulting drawing up of fluid into the second fluid conduit 38 for filling the dosing chamber 20 through the open non-return valve 31 due to the O-ring 30 being in the backward, non-sealing position.

In Figure 2, a dispensement is occurring - the non-return valve 31 is closed and the upward arrow is indicating that the main housing 12 is being moved towards the nozzle to force/pump the fluid out of the dosing chamber 20 up through the first fluid conduit 34. Of course, the piston 14 and nozzle 16 could be moved towards the housing 12 which is held static, or by simultaneously moving the piston 14 - nozzle 16 arrangement and the housing 12 towards each other.

In Figure 3, the arrow indicates the final moment of relative upward force against the main housing 12, whereupon the dispensement is completed - the piston member 14 has been fully displaced into the dosing chamber 20 so as to abut with the bottom wall 40 of the dosing chamber 20.

As the first annular groove 24 is spaced farther from the circular groove 36 than the second annular groove 26, it seals the top end of the gap between the side wall of the piston member 14 and the side wall of the dosing chamber 20. This stops fluid from leaking out of the dosing chamber 20 down the side of the piston member 14, and also prevents outside air from entering the device. The first O-ring 28 does not move significantly in the first annular groove 24. Indeed, the first annular groove 24 is less wide than the second annular groove 26. This will mean that the first O-ring 28 fits tightly within that first groove 24 once it is being compressed against both the side wall of the dosing chamber 20 and the base of the first annular groove 24. It will therefore provide a good, constant, seal between the piston member 14 and the side wall of the dosing chamber 20.

It is preferred that the source of fluid will be a bottle or receptacle onto which the main housing 14 is attached. It might be screwed onto the bottle. Alternatively, the arrangement of Figures 5 and 9 to 14 might be used.

The bottle may be vented, or may have some other configuration to prevent a back-pressure airlock as the fluid supply is used. For example the dispenser disclosed in WO-A-2005/075103 or WO-A-2004/014566 use a bottle which incorporates a piston in their bottles.

Preferably, the bottle is non-venting.

Referring to Figure 1 and the second end 42 of the piston member 14, the first fluid conduit 34 extends up through the piston member 14 and exits out of a side port 44 in a nipple 60 at the second end 42. The side port 44 is open to allow fluid within the dosing chamber 20 to be pumped from the dosing chamber 20, up through the first fluid conduit 34 and then into the fluid dispensement chamber 46 formed in the nozzle 16. The fluid dispensement chamber 46 occupies the entire upper internal space of the nozzle 16 and comprises two cylindrical portions. The first portion - the upper cylindrical portion - is sized to loosely receive the nipple 60 of the piston member 14. The second portion - the lower cylindrical portion - serves to receive a piston arrangement provided at the second end 42 of the piston member (much like the previously described piston arrangement at the first end 22 of the piston member 14).

The diameter of the lower cylindrical portion is larger than the diameter of the dosing chamber 20. The diameter of the piston arrangement at the second end 42 of the piston member 14 is therefore larger than the piston arrangement at the first end 22 of the piston member 14.

The lower cylindrical portion has a constant cross-section, and that cross-section continues down to the bottom of the nozzle 16.

The piston arrangement at the second end 42 of the piston member 14 is located within the lower cylindrical portion. The piston arrangement comprises a substantially cylindrical portion having a groove with an O-ring 48 in it. The O-ring 48 seals that piston against the side wall of that lower cylindrical portion.

In place of the O-ring 48, an integral resilient member might be provided, for example one that is moulded onto the piston member 14. Some other known sealing means might alternatively be used.

This second piston (within the lower cylindrical portion of the nozzle 16) serves to pressurise fluid in the fluid dispensement chamber 46.

The nipple 60 of the piston member 14 extends away from the lower cylindrical portion, to be located, in use, in the upper cylindrical portion of the nozzle 16 and has a generally loose fit whereby fluid can pass around its outer surface.

The upper cylindrical portion of the nozzle 16 has an end wall defining the top of the fluid dispensement chamber 46. A fluid outlet 52 is provided in that top, through which the pressurised fluid from the fluid dispensement chamber 46 can exit the nozzle 16 for dispensement to a user, e.g. in the form of a spray as shown in Figure 2. The dispensement may be for delivery to a nostril of the user.

The fluid outlet 52 is associated with a sealing member 54 in the form of a further O-ring 54. The O-ring 54 is significantly smaller than the previous O-rings and it forms part of a seal for closing the fluid outlet 52. The other part of that seal is an end wall 50 of the nipple 60.

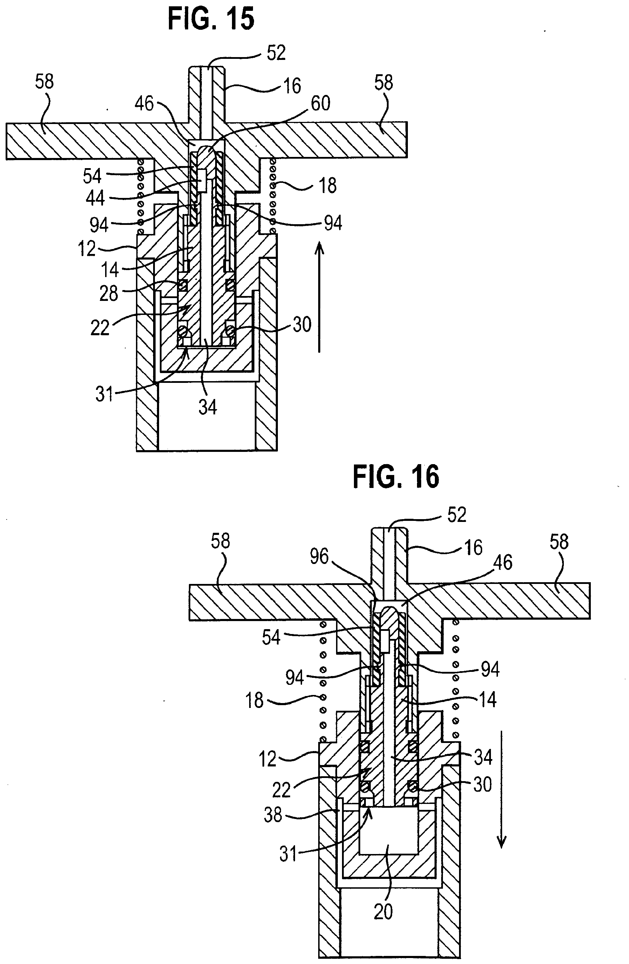

The end wall 50 of the nipple 60 has a rounded tip. When the end wall 50 is pushed against the sealing member 54, i.e. with the rounded tip in the middle of the O-ring 54, the seal will be closed. This occurs in the default or rest position of the dispenser 10, as shown in Figure 1. It occurs because the end wall 50 is biased into engagement with the sealing member 54 by virtue of the spring 18 biasing the piston member 14 into a position spaced from the bottom wall 40 of the dosing chamber 20 (Figure 1). It will be appreciated that the nozzle 16 is restrained from moving beyond the position shown in Figure 1 to ensure that the biasing force has this desired effect. For example, there are clips 86 and grooves in the embodiment of Figure 5. However, the restraining mechanism might be some known mechanism, such as an outer casing against an inside surface of which a flange 58 (see Figure 15) of the nozzle 16 may bear.

To dispense fluid from the dispenser 10, the nozzle 16 needs to be compressed relative to the main housing 14, as shown in Figures 1 and 2. The compression of the nozzle 16 will drive the piston member 14 into the dosing chamber 20 through the interengaging surfaces of the nozzle 16 and the piston member 14. This causes compression of the spring 18. During an initial phase of this movement, the end wall 50 of the piston member 14 will stay engaged to the sealing member 54 to keep the outlet 52 closed. During this initial bleed phase, the piston 14 pumps the surplus volume of fluid in the dosing chamber 20 through the entrance hole back into the second fluid conduit 38, as described above. However, once the closed non-return valve 31 on the piston member 14 passes the entrance point for the second fluid conduit 38, to therefore define the metered volume of fluid in the dosing chamber 20, continued downward movement of the piston member 14 relative to the dosing chamber 20 causes pressure to build up within the fluid in front of the first end 22 of the piston member 14, since the fluid will no longer be able to bleed through the entrance point due to the non-return valve 31 being closed, nor exit through the outlet 52 as the seal member 54 still sealingly co-operates with the nipple end wall 50.

Once the build-up of fluid pressure is enough, there will be a force on the piston member 14 that is sufficient to overcome the biasing force provided by the spring 18 against the piston member 14. As shown in Figure 1, that force causes the piston member 14, and hence the end wall 50 of the nipple 60, to move away from the sealing member 54, thereby opening the fluid outlet 52. The pressurised fluid will then be free to escape out of the fluid dispenser 10 through the fluid outlet 52, and since it is pressurised by the pumping action of the piston member 14, it will exit the fluid dispenser 10 as an atomised spray. Further, that spraying will continue while the relative compression of the piston member 14 and dosing chamber 20 is continued until a) the piston member 14 is completely pressed into the dosing chamber 20 to abut the end wall 40, and b) the piston member's end wall 50 has resealed the fluid outlet 52 by re-engaging the sealing member 54 due to the nozzle 16 continuing to move relatively downwardly, which movement is now also relative to the piston member 14 due to its abutment with the dosing chamber end wall 40. The fluid dispenser 10 has then been fully compressed, as shown in Figure 3.

As will be understood, the piston configuration at the second end 42 of the piston member 14 and the fluid dispensement chamber 46 are configured and arranged so that, when the fluid in front of the first end 22 of the piston member 14 is pressurised once the non-return valve 31 closes the entrance point to the dosing chamber 20, the pressurised fluid acts to separate the nozzle 16 and the piston member 14 to open the fluid outlet 52.

Once fully compressed, the fluid dispenser 10 can be released, whereupon the spring 18 will apply a return force against the interengaging nozzle 16 and the piston member 14 for resetting the fluid dispenser 10 to the configuration shown in Figure 1, during which resetting the nozzle 16 and the piston member 14 will again be separated from the main housing 12. During that motion, the one-way valve 31 will open, as described hereinabove, whereupon fluid will be drawn into the dosing chamber 20 from the source of fluid until the default position of Figure 1 is reached. In more detail, as the piston member 14 moves from the Figure 3 configuration back to the Figure 1 configuration, as illustrated in Figure 4, a negative pressure is created in the dosing chamber 20 which draws fluid from the fluid source along the conduit 38, through the entrance point and into the dosing chamber 20 through the open non-return valve 31. Once the open non-return valve 31 passes the entrance point, the fluid continues to fill the expanding dosing chamber 20 directly. That then completes the product's use cycle, ready for its next dispensement, with the dosing chamber 20 once more overfilled with fluid.

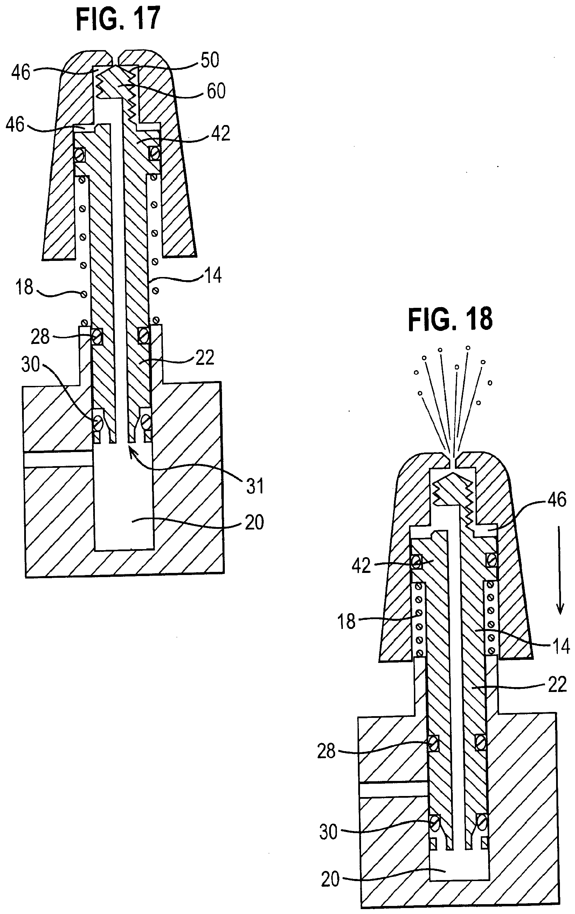

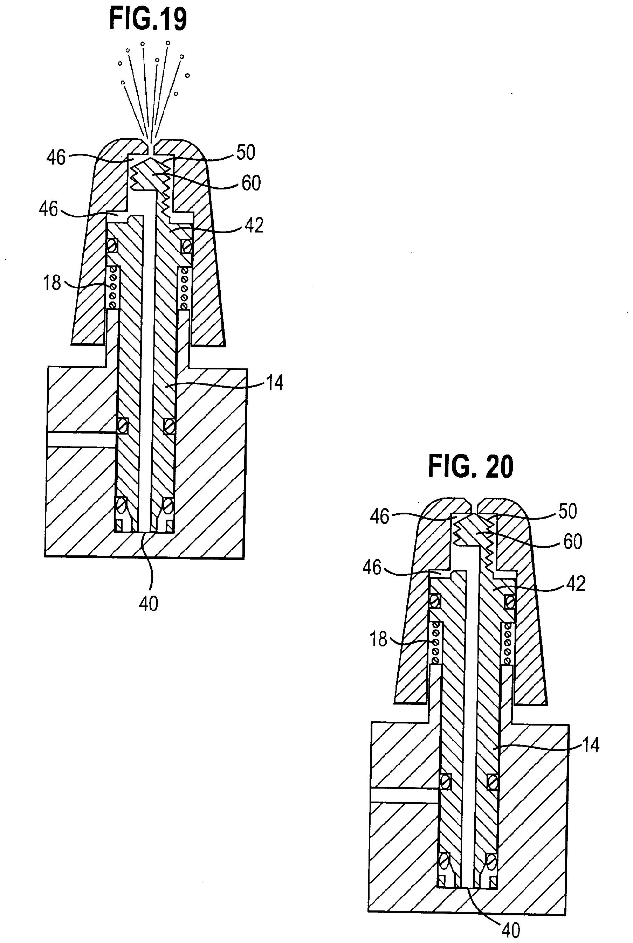

Referring now to Figures 17 to 20, a similar arrangement for a fluid dispenser 10 to that of Figures 1 to 4 is shown. However, in this embodiment, predefined fluid flow paths are provided between the side wall of the upper cylindrical portion of the nozzle 16 and the side wall of the nipple 60 by a screw thread on the side wall of the nipple 60. Flow paths for fluid past the nipple 60 in the embodiment of Figures 1 to 4 were instead provided just by a thin space between the side wall of the nipple 60 and the side wall of the upper cylindrical portion.

It will be appreciated that longitudinal grooves might instead be provided.

The operation of the fluid dispenser 10 of Figures 17 to 20 is substantially identical to the operation of the device of Figures 1 to 4. However, a further structural difference exists in that the ramp in the second annular groove 26 is angular in this alternative embodiment. It has a single landing, as before. However, it has a straight ramp, rather than a curved ramp.

Referring now to Figures 5 to 8, another alternative arrangement is disclosed which operates to the same principle as the embodiment of Figures 1 to 4. This arrangement again comprises the non-return valve 31 at the first end 22 of the piston member 14, a spring 18 and a nozzle 16. However, a nozzle cap 62 surrounds the nozzle 16.

The nozzle cap 62 is press-fitted onto the nozzle 16, and is removable for hygiene reasons. It can grip onto the nozzle 16 since the nozzle 16 has a flat shoulder 64 and a neck at its top end, which shoulder 64 and neck is adapted to fit with a corresponding shoulder 66 and hole in the top of the nozzle cap 62.

The nozzle cap 62 has flanges 58 for allowing the nozzle cap 62, and hence also the nozzle 16, to be compressed down relative to the main housing 12. That, as before, will cause the dispensement of fluid from the dosing chamber 20, up through the fluid conduit 34, out of a side port 44, into a fluid dispensement chamber 46 and out of a fluid outlet 52 after passing through an open seal. However, in this embodiment there are two side ports 44 - one on either side of the nipple 60. Further, the sealing member 54 is now a resilient or flexible tab or plate (e.g. made of rubber or silicone). That plate 54 is for closing over the hole of the fluid outlet 52 when pressed into a sealing position by the end wall 50 of the nipple 60.

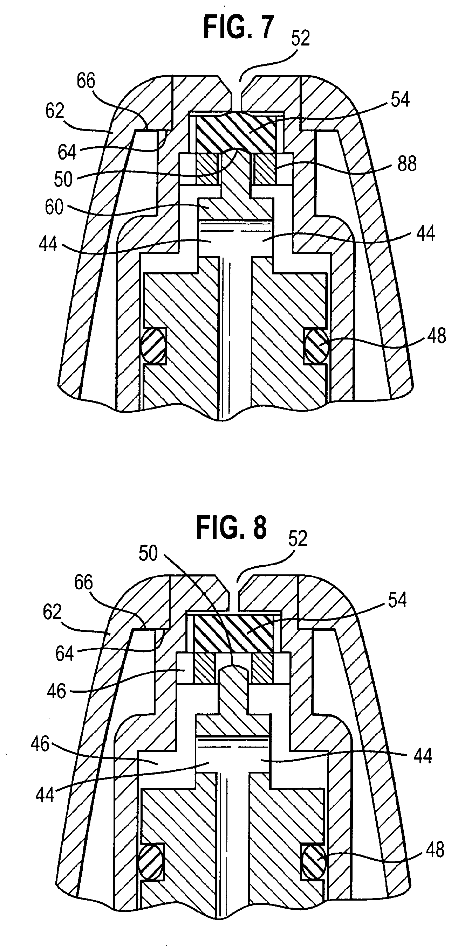

The sealing member 54 is shown in more detail in Figures 7 and 8. It can be flexed by the end wall 50 into a sealing position by the pressing or engagement of that end wall 50 against an underside of the sealing member 54. That sealed position is the default or rest position - see Figure 7. However, as before, the piston configuration at the second end 42 of the piston member 14 and the fluid dispensement chamber 46 are configured and arranged so that, when the fluid in front of the first end 22 of the piston member 14 is pressurised once the closed non-return valve 31 closes the entrance point to the dosing chamber 20, the end wall 50 disengages from the sealing member 54 whereupon¹ the sealing member 54 will be free to relax into a substantially flat shape - see Figure 8. In that flat state, fluid can exit the fluid dispensement chamber 46 by escaping over the top surface of the sealing member 54, thereby reaching and exiting through the fluid outlet 52. Alternatively, the pressurised fluid itself flattens the sealing member 54 after the end wall 50 disengages therefrom.

The sealing member 54 comprises on an underside of it a spacer and centralising member 88. That member 88 is a ring of material and it can be either stiff or flexible. The tip of the nipple 60 fits within the middle of that ring of material to ensure that the end wall 50 pushes against the middle of the sealing member 54 so as properly to close the seal.

The main housing 12 takes the form of a thin-walled, U-shaped cylindrical element having two opposing holes 68 through its side wall. Those holes 68 are the entrance points for the second fluid conduit 38 of this embodiment.

The dosing chamber 20 is defined by the interior of the thin-walled, U-shaped cylindrical element 12. The second fluid conduit 38 is the annular gap surrounding the main housing 12, between that main housing 12 and a stopper portion 76. It is capped by an outwardly extending flange provided around the circumference of the main housing 12. That flange is preferably welded onto the stopper portion 76 for that purpose, although some other seal might be provided.

The second fluid conduit 38, as before, allows fluid to be fed from a bottle 70 into the dosing chamber 20. In this embodiment, however, a supply or dip tube 72 is provided, which supply tube 72 extends from the end of the fluid conduit 38 to adjacent the bottom of the bottle 70, whereby an upright bottle 70 can still supply the fluid even when the bottle is nearly empty.

In this embodiment, a lesser degree of overfill occurs (compare Figures 1 and 5 - in Figure 5, the non-return valve 31 only slightly rises above the entrance point 68 for the second fluid conduit 38). More overfill might, however, be provided, if desired, by moving the opposing holes 68 down.

The stopper portion 76 is adapted to be pushed inside the neck 78 of the bottle 70 like a cork. That arrangement is then secured in place on the bottle 70 by a sealing cap or ferrule 74. That sealing cap 74 tightly grips the stopper portion 76 onto the neck 78 by overlying a flange 80 of the neck 78.

The stopper portion 76 additionally comprises its own neck portion 82. That neck portion 82 has two opposed grooves 84 in it. Those grooves 84 extend generally axially, i.e. parallel to the piston member 14, along a portion of the neck portion 82.

The side walls of the nozzle 16 fit into the neck portion 82. However, to lock it in place, i.e. to prevent the nozzle 16 from extending away from the main housing 12 beyond the position shown in Figure 5, two clips 86 are provided on the outside wall of the nozzle 16. They engage into the grooves 84.

In use, as in the embodiment of Figures 1 to 4, the spring 18 causes the fluid dispenser 10 to take a fully extended default/rest position, as shown in Figure 5. Then, upon relative compressing of the nozzle 16 towards the main housing 12, the biasing force of the spring 18 maintains the piston member 14 against the sealing member 54 until such time that the O-ring 30 of the non-return valve 31 passes the two entrance holes 68 in the side walls of the dosing chamber 20. Thereafter, the fluid (hydraulic) pressure will start to build-up in the fluid dispensement chamber 46, as before. That pressure will then eventually cause the piston member's end wall 50 to separate from the sealing member 54. That will allow the seal 54 to open, whereafter the metered dose of pressurised fluid will start to dispense out of the fluid dispenser 10 through the fluid outlet 52 (see Figure 6).

That dispensement will then continue until the piston member 14 hits the bottom wall 40 of the dosing chamber 20 and the nozzle 16 then moves relative to the piston member 14 until the piston member 14 re-engages with the sealing member 54 to close the seal 54 for the fluid outlet 52.

After the dispensement, the mechanism can be released to return itself to the start position (as in Figure 5), during which time the dosing chamber 20 will be recharged with a surplus of fresh fluid from the bottle 70 via the second fluid conduit 38 and the supply tube 72.

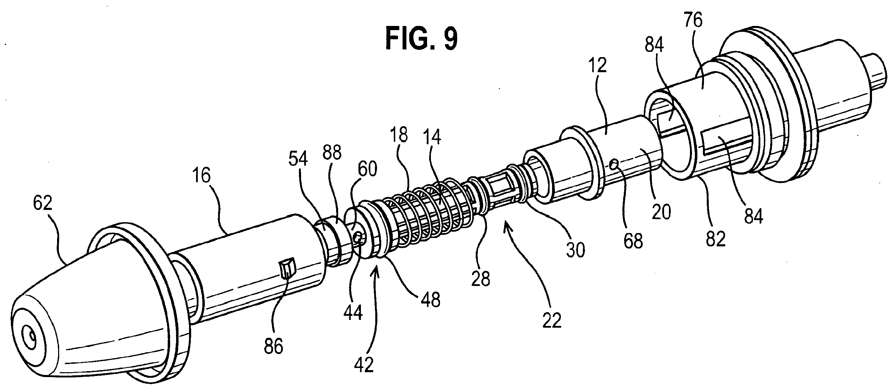

Referring now to Figures 9 to 14, a method of assembling the fluid dispenser of Figures 5 to 8 is illustrated.

The fluid dispenser 10 comprises, from left to right in Figure 9, the nozzle cap 62, the nozzle 16, the sealing member 54, the spacer and centralising member 88, the piston member 14 (with its three O-rings 28, 30, 48), the spring 18, the main housing 12 and the stopper portion 76.

The stopper portion 76 has the two grooves 84 and the neck portion 82.

The main body 12 has its dosing chamber 20 inside it and the holes 68 in the side wall of that dosing chamber 20 (only one of those holes 68 is visible).

The two ends 22, 42 of the piston member 14 each have a fixed O-ring 28, 48 positioned in its appropriate groove 24. Further, the O-ring 30 for the one-way valve 31 is located in its groove 26.

The piston member 14 has its nipple 60 facing away from the dosing chamber 20 (just one of the two side ports 44 is visible in that nipple 60).

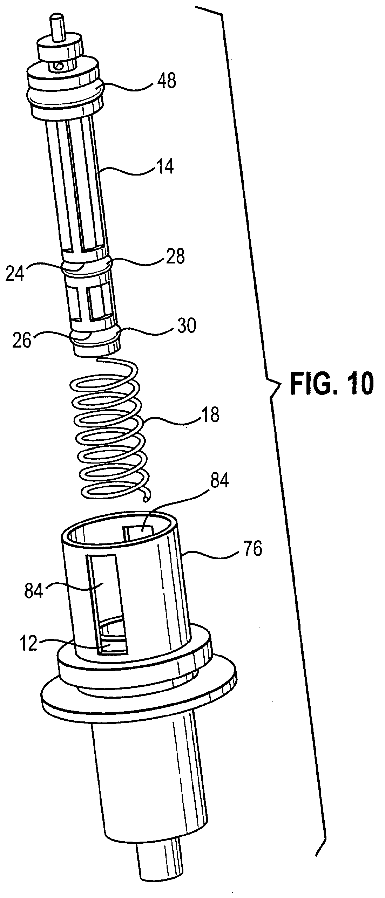

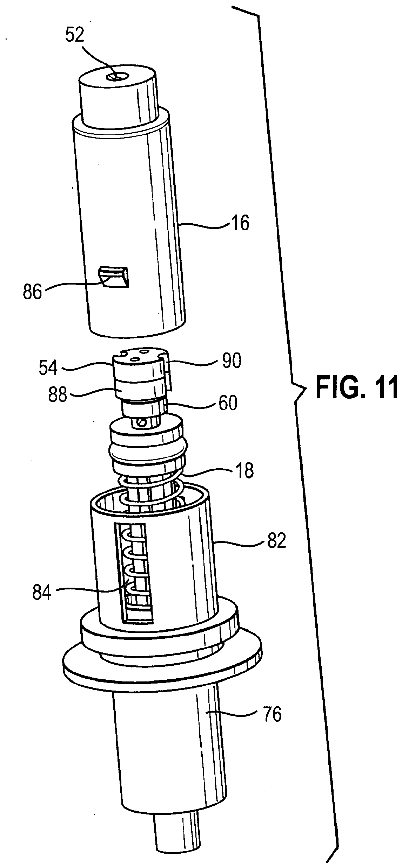

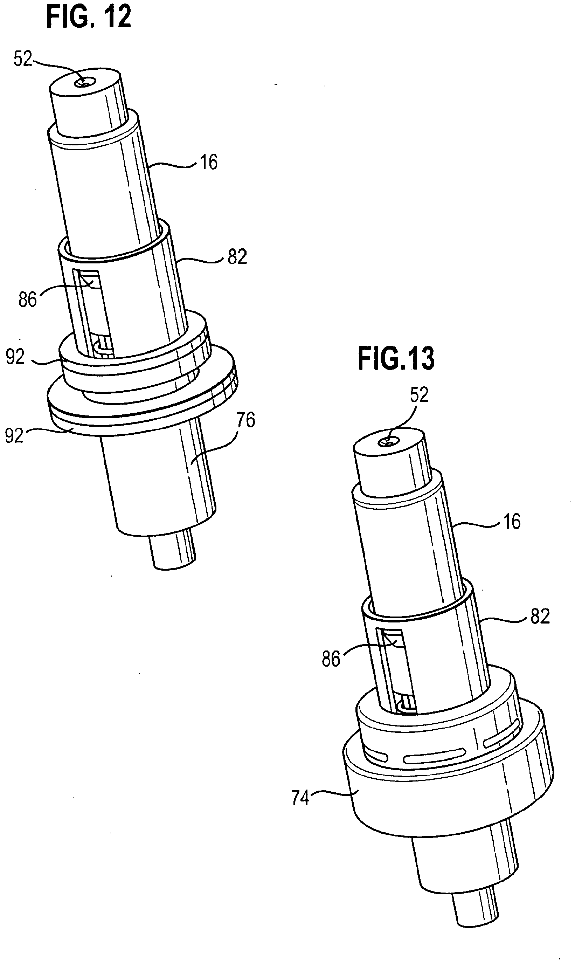

The preferred order for assembly requires the main housing 12 to be slotted into the stopper portion 76. It may then be ultrasonically welded in position to form a hermetic seal between the two elements, whereby the second fluid conduit 38 is formed. Then the spring 18 and the piston member 14, with its three O-rings 28, 30, 48, are inserted into the main housing 12, as shown in Figure 10. Then the sealing member 54, with its attached spacer and centralising member 88, is put onto the nipple 60 of the piston member 14. Then the nozzle 16 is snapped over that arrangement, engaging its two clips 86 (one shown) into the grooves 84 of the neck portion 82 of the stopper portion 76. This assembly step is shown in Figure 11. The resulting arrangement is shown in Figure 12.

The nipple 60 in Figure 11 has an larger outer diameter than as shown in Figures 5 to 8, indicating a possible modification of the nipple 60.

Returning back to Figure 11, both the sealing member 54 and its attached spacer and centralising member 88 have a fluid flow groove 90 on at least one side thereof. That groove 90 is provided to improve fluid flow past them during fluid dispensement.

Referring again to Figure 12, two further seals 92 are then positioned onto the resulting arrangement. Those seals 92 are for sealing with the sealing cap 74 and the bottle neck 78, respectively, i.e. once that arrangement is finally mounted and secured onto the neck 78 of the bottle 70. The lower seal 92 is positioned on an annular flange extending outwardly from the stopper portion 76. The seals 92 and the annular flange are not shown in Figures 5 and 6, but their intended location in Figures 5 and 6 will be understood.

Once assembled onto the bottle, the nozzle cap 62 is pushed onto the nozzle 16 to complete the assembly, although that could have been done earlier.

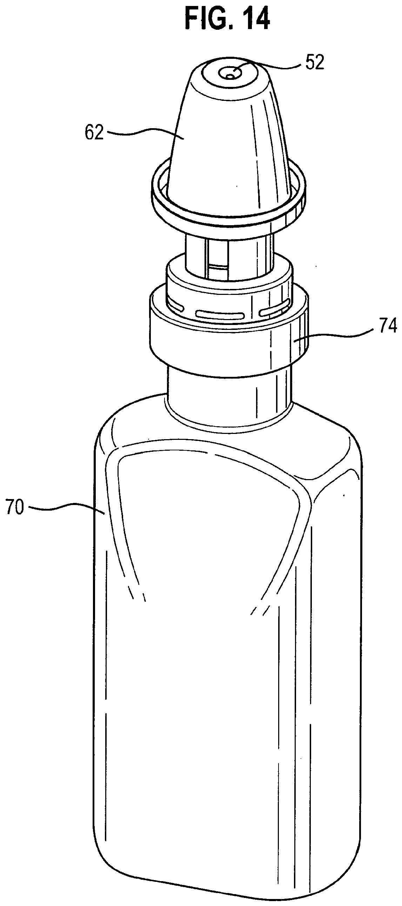

The completed assembly is shown in Figure 14, with Figures 5 and 6 showing the completed assembly in section.

Referring now to Figures 15 and 16, a further alternative sealing arrangement. In this embodiment, in place of a sealing member at the fluid outlet, the sealing member 54 seals the side port 44 for the fluid conduit 34 that is provided in the nipple 60 of the piston member 14.

The sealing member 54 is a resilient tube that is held onto the nipple 60 by the resilience of the tube. In this preferred arrangement, that securement is assisted by two clips 94.

As in the previous embodiments, a non-return valve 31 is provided at the first end 22 of the piston member 14. Further, the general principle of refilling of the dosing chamber 20 is no different to before. The arrangement of the fluid conduit 34 in the piston member 14 is also unchanged. However, whereas before a large fluid dispensement chamber 46 was provided, in this embodiment a significantly smaller fluid dispensement chamber 46 is provided - the larger cross-sectional area is no longer required.

In order to dispense fluid from this device, the fluid pressure again needs to be raised in order to open the seal 54. In this case, however, it is the hoop stress within the resilient tube that needs to be overcome. That is achieved, as before, by relative compressing of the nozzle 16 towards the main housing 12. That compression, once the non-return valve 31 has passed the entrance point for the second fluid conduit 38, still causes the fluid pressure to build up and that built-up pressure will eventually overcome the hoop stress in the resilient tube 54, whereupon the tube 54 will expand away from the nipple 60. Only then will pressurised fluid escape into the fluid dispensement chamber 46 for dispensement out through the fluid outlet 52.

In order for there to be space for that expansion of the resilient tube, a narrow gap 96 (see Figure 16) is provided between the sealing member 54 (the resilient tube) and an internal wall of the fluid dispensement chamber 46. By keeping the gap narrow, the fluid will have a greater tendency to spray out through the fluid outlet 52.

A spring 18 may again be provided in this embodiment, as shown. However, it just serves to bias the nozzle 16 away from the main body 12.

As previously described, flanges 58 are provided in this embodiment. They additionally, however, allow the nozzle 16 to be grasped by the user for relative compressing of the nozzle 16 down against the main housing 12.

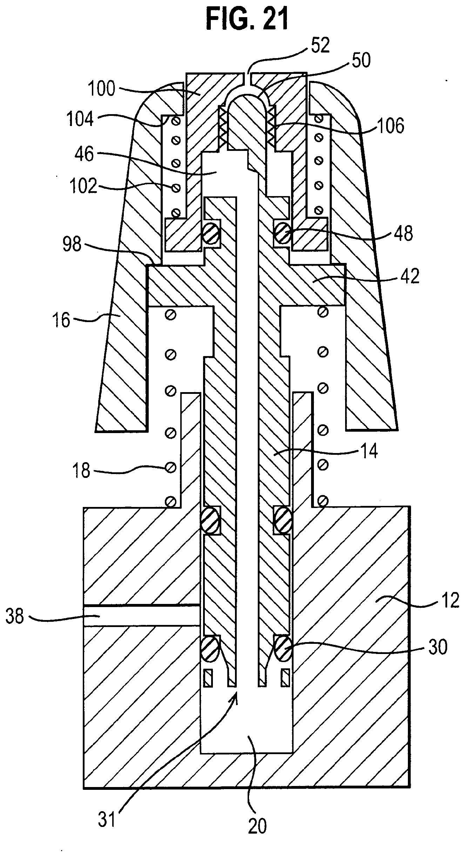

Finally, referring to Figure 21, a further embodiment is disclosed having the same general operating principle on the embodiment of Figures 1 to 4. In this embodiment, the non-return valve 31 is again provided at the first end 22 of the piston member 14. Further, the spring 18 is provided to bias the piston member 14 and nozzle 16 away from the main housing 12 for the purpose of filling the dosing chamber 20. However, this embodiment has a different nozzle arrangement.

The nozzle 16 comprises a hollow main body and a separate nozzle component 100 fitted therein. The hollow of the main body is cylindrical, but with a shoulder 98 approximately half way along it, which shoulder 98 separates a first and larger cylindrical portion from a smaller cylindrical portion. The smaller cylindrical portion is positioned towards the top of that main body, i.e. spaced farther from the main housing 12 of the fluid dispenser 10. The separate nozzle component 100 is located within that smaller cylindrical portion.

A flange extends around the circumference of the piston member 14 below the piston of the second end 42. That flange engages the underside of the shoulder 98 within the main body of the nozzle 16. Further, the spring 18 acts upon the underside of that flange, and also upon the top of the main housing 12, to bias that flange of the piston member 14 into engagement with that shoulder 98. That force holds the nozzle's main body and the piston member 14 together such that they will move in unison throughout the use cycle of the fluid dispenser 10, i.e. both during compression and release operations carried out on the fluid dispenser 10.

The separate nozzle component 100 of the nozzle 16 slidingly fits within the smaller cylindrical portion of the main body of the nozzle 16. It is also hollow. The hollow defines a) the upper cylindrical portion for the nipple 60 of the piston member 14 and b) the lower cylindrical portion for the piston at the second end 42 of the piston member 14.

The fluid outlet 52 is provided in the top of the hollow of the nozzle component 100. Further, the end wall 50 of the nipple 60 is adapted to seal that fluid outlet 52. In this embodiment, the end wall 50 of the nipple 60 is rubberised for sealing that fluid outlet. However, the previously disclosed O-ring or sealing plate from the earlier embodiments would also work.

In a similar fashion to the embodiment of Figure 17, the upper cylindrical portion of the separate nozzle component 100 has predefined fluid flow paths around the nipple 60. They are again spiral channels, but this time are provided as a separate, folded or coiled member. However, it could also be formed integrally with the nozzle component 100, for instance as a screw thread profile.

The piston at the second end 42 of the piston member 14, as before, includes an O-ring 48. It now, however, provides a sealing fit within the lower cylindrical portion of the separate nozzle component 100. That sealing fit closes the bottom of the fluid dispensement chamber 46, which is now within the separate nozzle component 100. That fluid dispensement chamber 46, however, can be fed pressurised fluid from the dosing chamber 20 in much the same way as in the previous embodiments, i.e. via a fluid conduit 34 that extends through the piston member 14 and out through a side port in the nipple 60.

In this embodiment, the lower cylindrical portion has the same diameter as the dosing chamber 20. A different mechanism for opening the seal for dispensement through the fluid outlet 52 is therefore needed. In this embodiment it is provided by the provision of a biasing means (a spring 102) between the nozzle's main body and the separate nozzle component 100. That spring 102 fits between the nozzle's main body and the separate nozzle component 100. It engages both a flange provided around the bottom perimeter of the separate nozzle component 100 and a second flange provided around the inside of the top of the smaller cylindrical portion of the nozzle's main body. The spring 102 therefore biases the separate nozzle component 100 downwards relative to the main body of the nozzle 16, i.e. onto the end wall 50 of the piston member 14. It therefore causes the fluid outlet 52 to be sealed closed by default. However, pressure build-up in the fluid of the fluid dispenser 10 during the dispensing part of the actuation cycle will eventually overcome the biasing force of the spring 102 and separate the nozzle component 100 from the nipple 60, in this particular embodiment by moving the nozzle component 100 upwardly, away from the nipple 60. By overcoming the biasing force of the return spring 102, the seal will be opened, whereupon fluid dispensement can occur through the fluid outlet 52.

By varying the return force of the spring 102, i.e. by using weaker or stronger springs, different pressures will be required to open the seal. If a large force is required, the fluid will be under a greater pressure at the time of dispensement. That may be advantageous for forming a powerful spray. However, the compression force necessary to overcome that spring force must be within the abilities of a user.

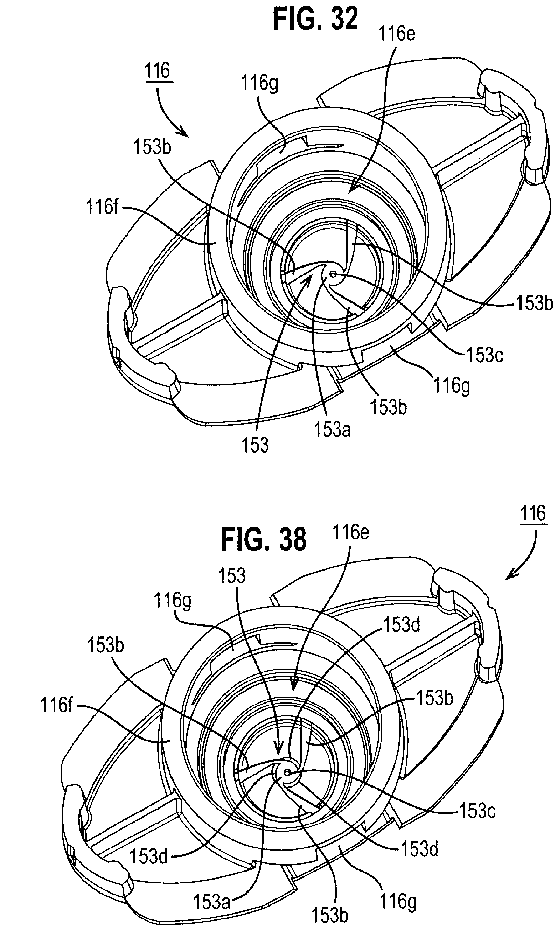

Each of the afore-described fluid dispensers may be provided with a swirl chamber at the fluid outlet, as will be understood by the skilled person in the art. For instance, the swirl chamber 153 illustrated in Figures 32 and 38 could be employed.

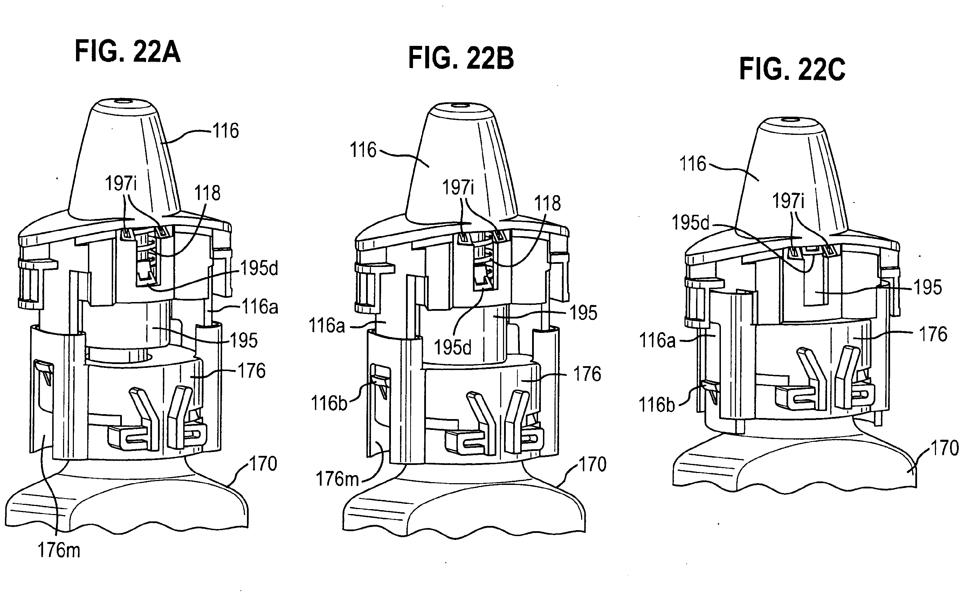

Figures 22 to 36 show a fluid dispenser in accordance with the present invention with those features which are like features in the previously described fluid dispensers of Figures 1 to 21 being indicated by like reference numerals.

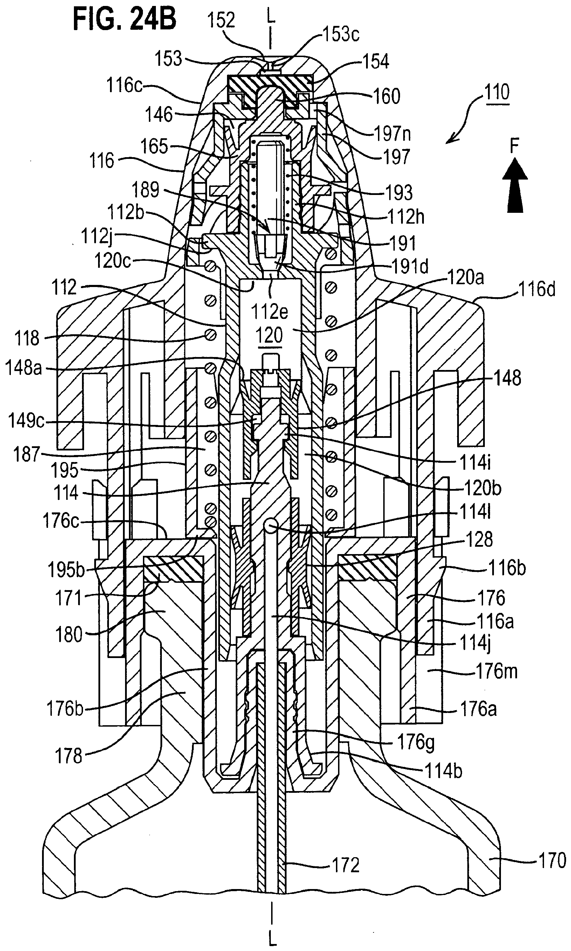

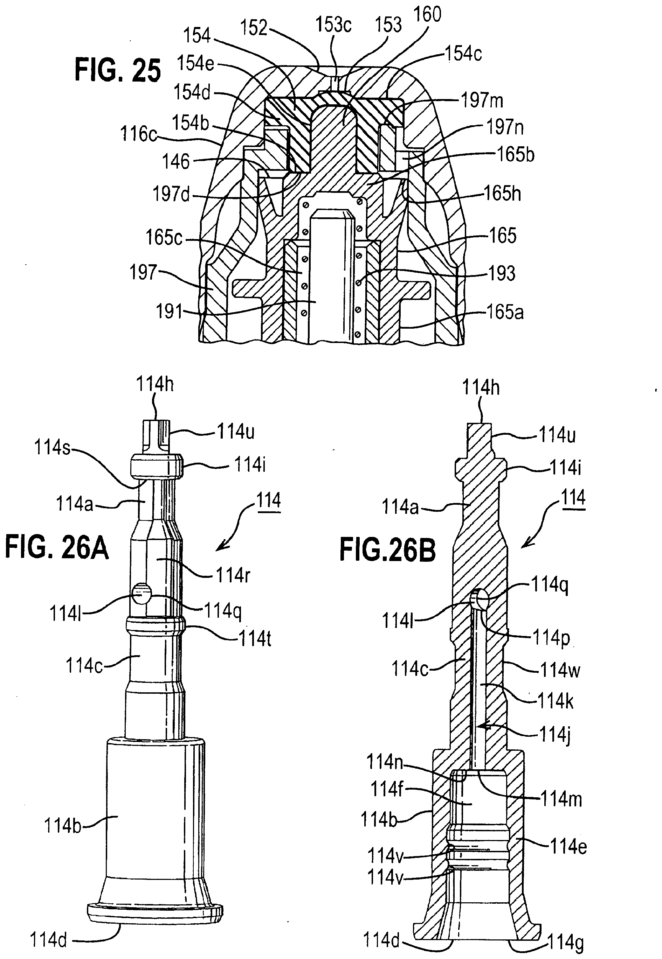

Referring to Figures 24B, 26A and 26B, the piston member 114 of the additional fluid dispenser has a generally cylindrical form and is mounted to stroke in reciprocal fashion along a longitudinal axis L-L of the fluid dispenser 110 inside the dosing chamber 120 defined by the main housing 112. The piston member 114 is mounted to stroke between forward and rear positions relative to the dosing chamber 120.

The piston member 114 in this embodiment is injection moulded from polypropylene (PP), but other functionally equivalent plastics materials could be used.

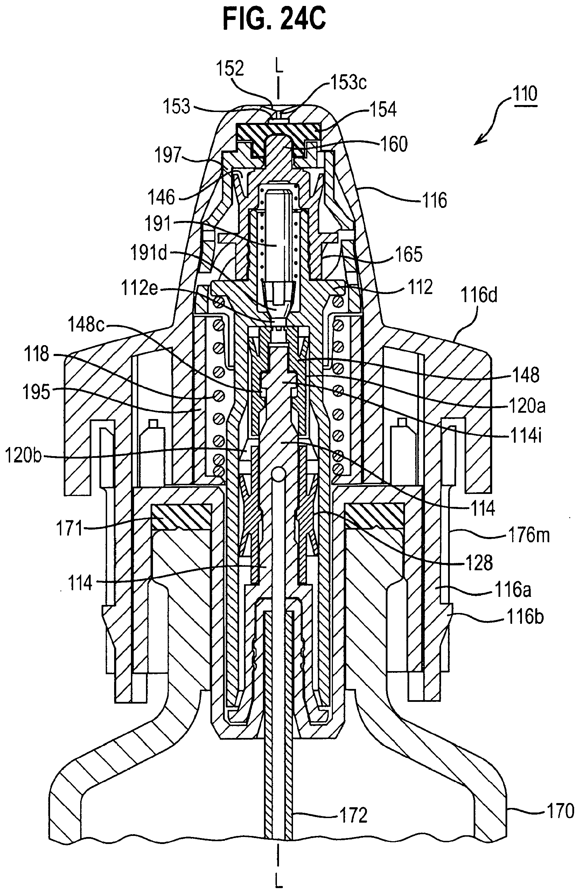

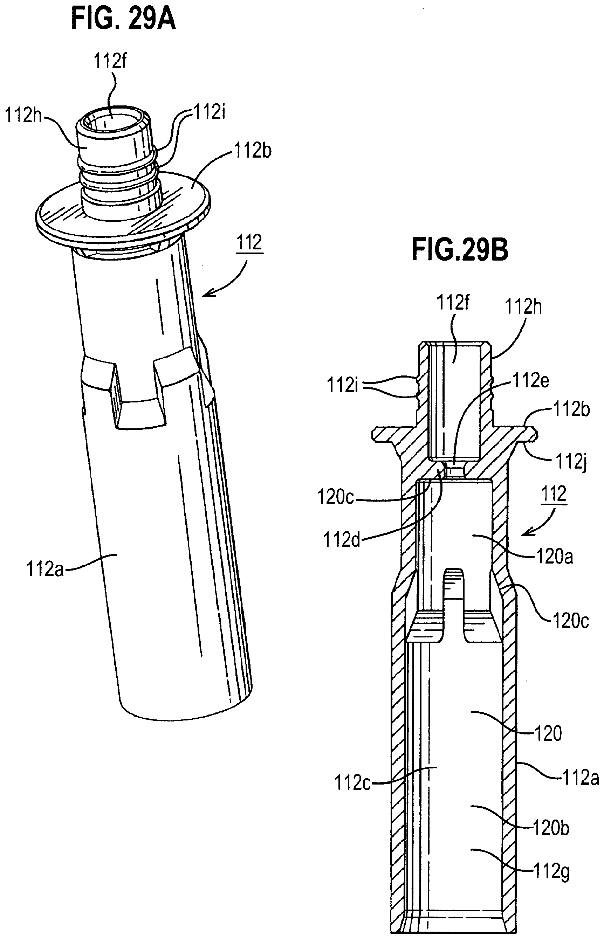

Referring to Figures 24B, 24C, 29A and 29B, the dosing chamber 120 is cylindrical and co-axially arranged with the longitudinal axis L-L. The dosing chamber 120 has forward and rear sections 120a, 120b. As can be seen, the forward section 120a is narrower than the rear section 120b. A step 120c tapers inwardly in the forward direction F (see Figure 24B) to connect the rear section 120b to the forward section 120a.

Turning back to Figures 26A and 26B, the piston member 114 has a forward section 114a, a rear section 114b and a central section 114c. These are arranged co-axially.

The rear section 114b presents the open rear end 114d of the piston member 114. The rear section 114b is cup-shaped having an annular outer peripheral wall 114e which defines an internal cavity 114f having a mouth 114g which opens in the rear end 114d.

The forward section 114a is solid and presents the forward end 114h of the piston member 114. The forward section 114a comprises an annular flange 114i rearwardly of the forward end 114h.

The central section 114c connects to the forward and rear ends 114a, 114b and comprises an internal bore network 114j to place the rear section 120b of the dosing chamber 120 in fluid communication with the fluid supply 170 (a bottle - see Figures 22A to 22C), as will be described in more detail hereinafter. The bore network 114j consists of an axial section 114k and plural transverse sections 114l. The axial bore section 114k extends forwardly from a rear opening 114m in a forward face 114n of the internal cavity 114f to a junction 114p. The transverse bore sections 114l extend transversely, inwardly from respective forward openings 114q in the outer circumferential surface of the central section 114c to the junction 114p to connect with the axial bore section 114k. The forward openings 114q are arranged equi-angularly about the central section 114c. In this particular embodiment, there are two transverse bore sections 114l, but one or greater than two transverse bore sections could be used. The forward openings 114q are also recessed in the central section 114c.

The piston member 114 is provided with a plurality of axially-oriented grooves 114r about the outer periphery. The grooves 114r extend rearwardly from a rear surface 114s of the annular flange 114i in the forward section 114a to an annular rib 114t on the central section 114c rearward of the forward openings 114q of the internal bore network 114j. The grooves 114r are arranged so that at least a portion of the forward openings 114q are within the grooves 114r.

The tip part 114u of the forward section 114a of the piston member 114, which extends forwardly from the flange 114i to the forward end 114h, has a triangular cross-sectional shape, with the apexes being rounded.

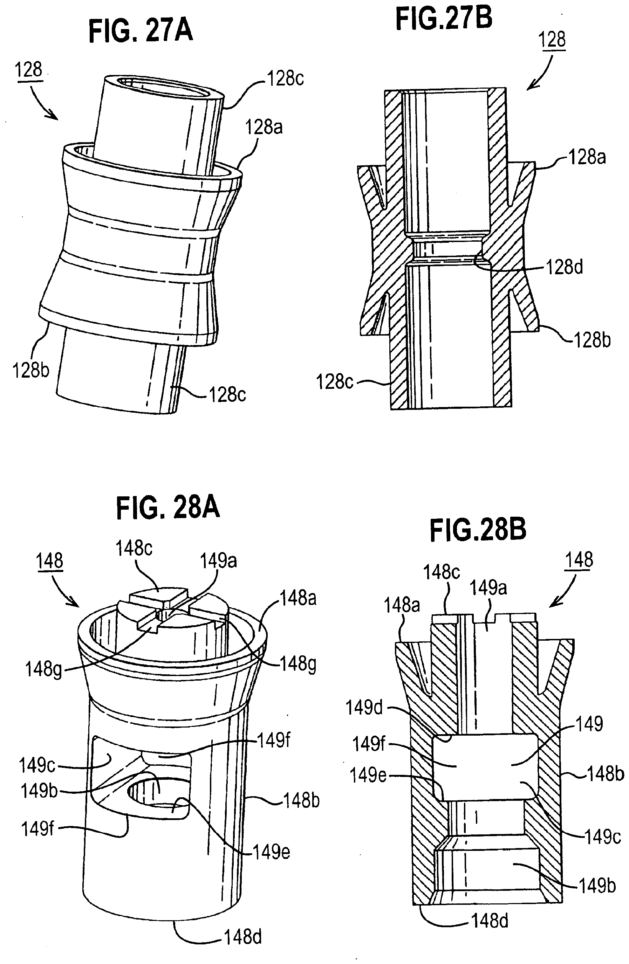

Referring to Figures 24B, 24C, 27A and 27B, the piston member 114 carries on its central section 114c a tubular rear sealing element 128 which provides a permanent dynamic (sliding) seal between the piston member 114 and the rear section 120b of the dosing chamber 120. The rear sealing element 128 is fixed to the piston member 114 to move in unison therewith so that there is no relative axial movement therebetween as the piston member 114 strokes in the dosing chamber 120.

The rear sealing element 128 is of the lip-seal type, being provided with resilient, annular sealing lips 128a, 128b at its forward and rear ends, respectively. The material of the rear sealing element 128 provides the sealing lips 128a, 128b with an inherent outwardly-directed bias. The sealing lips 128a, 128b have an outer diameter which is greater than the inner diameter of the rear dosing chamber section 120b, whereby the sealing lips 128a, 128b are compressed inwardly by the inner surface of the rear dosing chamber section 120b. As a result, the bias in the sealing lips 128a, 128b means they sealingly engage the inner surface of the rear dosing chamber section 120b.

The rear sealing element 128 further comprises a tubular body 128c from which the sealing lips 128a, 128b depend and which fits on the outer surface of the piston member central section 114c by engagement of an inner circumferential bead 128d of the rear sealing element 128 in a recessed portion 114w of the central section 114c of the piston member 114. The tubular body 128c has a length such that, when fitted on the piston member 114, it covers substantially the entire axial extent of the central section 114c of the piston member 114.

Now referring additionally to Figures 28A and 28B, the piston member 114 further carries on its forward section 114a a tubular forward sealing element 148 to form a dynamic (sliding) seal between the piston member 114 and the forward section 120a of the dosing chamber 120, but only during a particular phase of the piston member stroke, as will be described in more detail hereinafter.

The forward sealing element 148 is also of the lip-seal type, but this time only being provided with a resilient, annular sealing lip 148a at its forward end. The outer diameter of the forward lip seal 148a is less than the inner diameter of the rear dosing chamber section 120b, but greater than the inner diameter of the forward dosing chamber section 120a. Consequently, the forward sealing lip 148a is able to be biased into sealing engagement with the inner surface of the forward dosing chamber section 120a.

As will be observed, the forward sealing element 148 is slidably mounted on the forward section 114a of the piston member 114. In more detail, the forward sealing element 148 comprises a tubular body 148b, from which the sealing lip 148a depends, and provides an axial, open-ended bore 149 through the forward sealing element 148 in which the forward section 114a of the piston member 114 is slidably mounted. The bore 149 comprises forward and rear bore sections 149a, 149b and an enlarged, central chamber 149c. The forward and rear bore sections 149a, 149b respectively extend from the central chamber 149 to openings in the forward and rear ends 148c, 148d of the forward sealing element 148. The forward end 148c is provided with grooves 148g which intersect the forward bore opening therein. The central bore chamber 149c is provided with a pair of diametrically opposed windows 149f through the tubular body 148b.

The annular flange 114i of the piston member 114 is located inside of the central bore chamber 149c. The central bore chamber 149c has transversely-oriented forward and rear end walls 149d, 149e which selectively engage the annular flange 114i of the piston member 114 to delimit the sliding movement of the forward sealing element 148 on the piston member 114. Specifically, the forwardmost position of the forward sealing element 148 relative to the piston member 114 is delimited by the rear end wall 149e abutting the annular flange 114i, and conversely the rearmost position of the forward sealing element 148 relative to the piston member 114 is delimited by abutment of the forward end wall 149d with the annular flange 114i.

The sliding movement of the forward piston member section 114a in the forward sealing element bore 149 forms a one-way valve. The one-way valve is closed when the forward sealing element 148 is in its rearmost position relative to the piston member 114 and open as the forward sealing element 149 moves towards its forwardmost position relative to the piston member 114, as will be discussed in more detail hereinafter.

To this end, it will be understood that the annular flange 114i forms a fluid-tight seal against the forward end 149d of the central bore chamber 149c when the forward sealing element 148 is in its rearmost position.

In operation, as the piston member 114 strokes forwardly relative to the dosing chamber 120, the forward sealing element 148 moves forwardly with the piston member 114 through engagement of the annular flange 114i with the forward end wall 149d of the central bore chamber 149c. Thus, the one-way valve is closed in the forward stroke of the piston member 114. The forward stroke also brings the forward sealing element 148 into sliding sealing engagement with the forward section 120a of the dosing chamber 120.

Once the piston member 114 reaches its forward position at the end of its forward stroke, as delimited by abutment of the forward end 148c of the forward sealing element 148 with a forward end wall 120c of the dosing chamber 120, the piston member 114 starts its return, rearward stroke towards its rearward position. In an initial phase of the rearward stroke, the piston member 114 moves rearwardly relative to the forward sealing element 148 so that the one-way valve is moved to its open position for the rearward stroke. The rearward stroke of the piston member 114 ends with the piston member 114 being disposed in its rearward position, where the forward sealing element 148 is disposed in the rear dosing chamber section 120b so that the forward and rear dosing chamber sections 120a, 120b are in flow communication about the forward sealing element 148.

It will thus be appreciated that in an initial phase of the forward stroke of the piston member 114 in the dosing chamber 120, the piston member 114 moves forwardly relative to the forward sealing element 148 to (re)close the one-way valve.

The rear and forward sealing elements 128, 148 in this embodiment are injection moulded from low density polyethylene (LDPE), but other functionally equivalent plastics materials could be used.

The return, compression spring 118 in the fluid dispenser 110 is provided to bias the piston member 114 to its rearward (resting) position relative to the dosing chamber 120, which is shown in Figures 22B and 24B. The spring 118 may be made from a metal or a plastics material.

As shown in Figures 29A and 29B, the main housing 112 is formed by a tubular body 112a from which an annular flange 112b projects. The tubular body 112a has an open-ended axial bore 112c into which an annular shoulder 112d projects to create a restricted bore section 112e relative to the forward and rear bore sections 112f, 112g disposed on either side of the annular shoulder 112d. The rear bore section 112g defines the dosing chamber 120. The forward section 112h of the tubular body 112a is provided with a pair of circumferential beads 112i.

The main housing 112 in this embodiment is injection moulded from polypropylene (PP), but other plastics materials could be used.

The biasing force of the return spring 118 acts to reset the piston member 114 in its rear position relative to the dosing chamber 120 defined in the main housing 112 by acting on the main housing annular flange 112b to bias the main housing 112 forwardly to its relative position shown in Figures 22B and 24B.

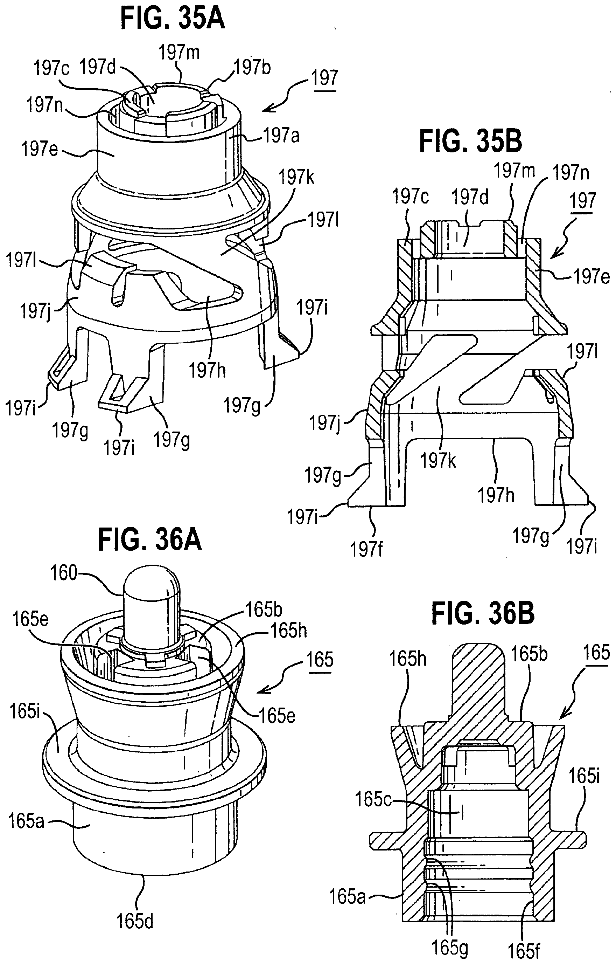

As shown in Figures 36A and 36B, the nipple 160 is comprised in a separate cylindrical cap 165. The cap 165 is of cup-form, having an annular side skirt 165a and a forward end wall 165b which form the boundary walls of an internal cylindrical chamber 165c which is open at the rear end 165d of the cap 165. Moreover, the nipple 160 is in the form of a central sealing tip which projects forwardly from the forward end wall 165b.

A plurality of apertures 165e are also formed in the forward end wall 165b, about the base of the sealing tip 160, to communicate with the internal chamber 165c. In this embodiment, there are three equi-angularly spaced apart apertures 165e, but alternatively there may be less or more in number than three apertures.

The inner circumferential side surface 165f of the internal chamber 165 is provided with a pair of circumferential beads 165g. The outer circumferential edge of the forward end wall 165b presents a resilient, annular sealing lip 165h.

In this embodiment, the cap 165 is formed from LDPE, but again other plastics materials could be used.

As shown in Figures 24B and 24C, for instance, the cap 165 is mounted over the forward section 112h of the main housing 112 to enclose the forward bore section 112f of the main housing 112. The cap 165 is secured to the main housing 112 by the respective internal and external beads 165g, 112i clipping or interlocking together such that they move in unison.

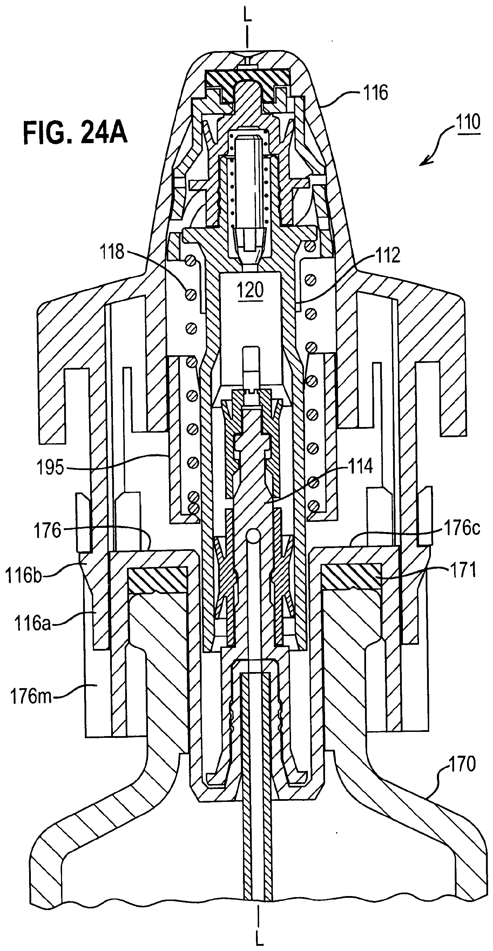

As further shown in Figures 24B and 24C, a valve mechanism 189 is located in the forward bore section 112f of the main housing 112. The valve mechanism 189 comprises a cylindrical, elongate valve element 191 mounted for axial movement in the forward bore section 112f.

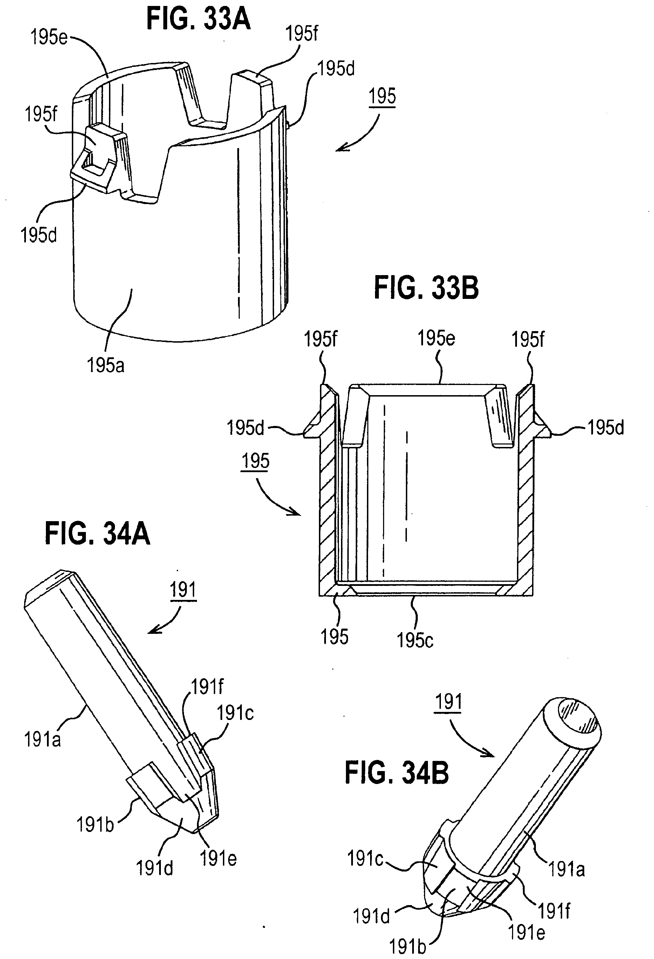

As shown in Figures 34A and 34B, the valve element 191 has a cylindrical forward section 191a and a coaxial, enlarged rear section 191b. The rear section 191b has a forward portion 191c and a frusto-conical rear portion 191d sized to sealingly fit in the restricted bore section 112e of the main housing 112 for closure thereof. A plurality of axial grooves 191e are formed in the outer peripheral surface of the rear section 191b to extend through the forward portion 191c and partially into the rear portion 191d.

Turning back to Figures 24B and 24C, the valve mechanism 189 further comprises a return, compression spring 193 which extends rearwardly from the inner surface of the forward end wall 165b of the cap 165 onto an annular flange 191 at the forward end of the rear section 191b of the valve element 191. The return spring 193 acts to bias the valve element 191 rearwardly to dispose the frusto-conical rear portion 191d in the restricted bore section 112e for sealing closure thereof.

The valve element 191 in this embodiment is injection moulded from low density polyethylene (LDPE), but other functionally equivalent plastics materials could be used. The return spring 193 may be of metal or a plastics material.

Figures 24B and 24C also show that the cylindrical stopper portion 176 has a cap form for fitting on the bottle neck 178. In this embodiment, the stopper portion 176 is injection moulded from polypropylene (PP). However, other plastics materials could be used.

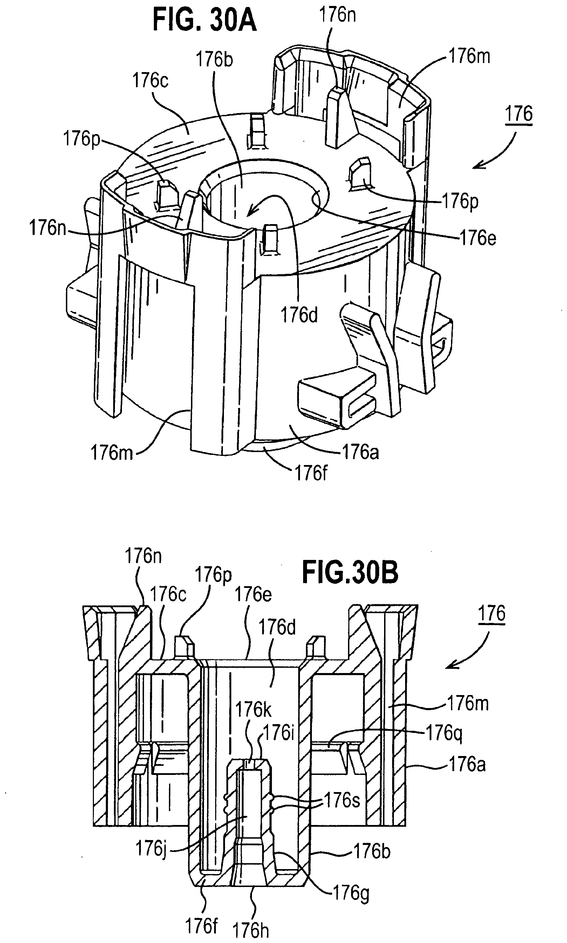

Referring also to Figures 30A and 30B, the stopper portion 176 has an outer annular skirt 176a, which surrounds the outer peripheral surface of the flange 180 of the bottle neck 178, and a concentrically arranged inner annular skirt 176b, which plugs the bottle neck 178. The inner peripheral surface of the outer annular skirt 176a is provided with circumferentially-oriented bead 176q to engage underneath the flange 180 of the bottle neck 178 to give a snap-fit connection of the stopper portion 176 to the bottle 170. The bead 176q may be continuous, or segmented to simplify the moulding of the stopper portion 176.

The stopper portion 176 has a roof 176c at its forward end extending radially inwardly from the outer skirt 176a to the inner skirt 176b. The inner skirt 176b encloses an internal cavity 176d which extends rearwardly from a opening 176e in the roof 176c. The cavity 176d has a floor 176f at its rear end from which upstands an elongate tubular projection 176g.

The tubular projection 176g has an open rear end 176h, a forward end wall 176i, an internal cavity 176j which extends forwardly from the open rear end 176h to the forward end wall 176i, and a forward opening 176k in the forward end wall 176i to place the internal cavities 176d, 176j in flow communication.

As shown in Figure 24B, for example, the supply tube 172 inserts into the internal cavity 176j of the tubular projection 176g as an interference fit, with the supply tube 176 abutting the forward end wall 176i of the tubular projection 176g. Likewise, the tubular projection 176g inserts into the internal cavity 114f of the rear section 114b of the piston member 114 so that the forward end wall 176i of the tubular projection 176g abuts the forward face 114n of the internal cavity 114f. In this way, the bore network 114j in the piston member 114 is placed in flow communication with the fluid supply 170 through the supply tube 172.

The tubular projection 176g is secured in the internal cavity 114f of the piston member 114 by the internal cavity 114f of the piston member 114 presenting a plurality of circumferential beads 114v on its inner circumferential surface to which clip or interlock circumferential beads 176s provided on the outer circumferential surface of the tubular projection 176g.

As further shown in Figure 24B, for example, the tubular body 112a of the main housing 112 is also mounted in the internal cavity 176d of the stopper portion 176 for relative sliding motion therebetween. The relative sliding motion between the stopper portion 176 and the main housing 112 effects the relative sliding motion between the piston member 114 and the dosing chamber 120 because the piston member 114 is carried on the tubular projection 176g of the stopper portion 176. The relative sliding motion is achievable by having the main housing 112 move rearwardly and maintaining the fluid supply 170 stationary, or vice-versa, or by having the main housing 112 and fluid supply 170 move towards one another at the same time.

It will be seen from Figure 24B, for example, that a sealing ring 171 is interposed between the stopper portion 176 and the fluid supply 170 to prevent leaks therebetween.

The fluid dispenser 110 further comprises a cylindrical carrier member 195 which surrounds the tubular body 112a of the main housing 112. As shown in Figures 33A and 33B, the carrier member 195 has an annular body 195a which is spaced radially outwardly of the tubular body 112a of the main housing 112 to define an annular space 187 therebetween (see Figure 24A). The annular body 195a has an inwardly projecting, annular flange 195b at its rear end 195c, and a plurality of outwardly projecting clips 195d disposed on tongues 195f defined by the castellated profile at its forward end 195e.

As shown in Figure 24B, the return spring 118 extends rearwardly from the rear face 112j of the main housing annular flange 112b into the annular space 187 between the carrier member 195 and the main housing 112 and onto the carrier member annular flange 195b for carriage thereon.

In normal use of the fluid dispenser 110, the carrier member 195 seats on the roof 176c of the stopper portion 176, both in the rest and fired positions of the fluid dispenser 110 to be discussed hereinafter. This normal position for the carrier member 195 is shown in Figures 24B (rest) and 24C (fired).

The carrier member 195 in this embodiment is also injection moulded from polypropylene (PP), but other plastics materials may be used.

Referring back to Figures 30A and 30B which show the stopper portion 176, it will be seen that the roof 176c carries a pair of diametrically opposed main protrusions 176n and a series of minor protrusions 176p arranged equi-angularly about the roof opening 176e. The main protrusions 176n are adapted in use to act on the outer circumference of the carrier member 195 to centralise it with respect to the stopper portion 176 as the carrier member 195 is seated on the roof 176c. The minor protrusions 176p fit into complementary grooves (not shown) in the annular flange 195b of the carrier member 195 to correctly orient the carrier member 195 on the roof 176c so that the clips 195d will clip into T-shaped tracks 116g in the nozzle 116 to be described hereinafter. In a modification, not shown, there may be provided just two minor protrusions, each forming a radial extension from one of the main protrusions.

The fluid dispenser 110 also comprises a tubular nozzle insert 197 surrounding the cap 165 mounted on the forward section 112h of the main housing 112. Figures 35A and 35B show the nozzle insert 197 has a hollow body 197a which at its forward end 197b has an end wall 197c through which is provided a central aperture 197d. The body 197a comprises a first annular section 197e which extends rearwardly from the forward end wall 197c and has, about it rear end, an outer circumferential bead 197p. The rear end 197f of the nozzle insert body 197a is presented by a plurality of spaced-apart, rearwardly extending legs 197g. There are four legs 197g in this embodiment. The legs 197g are arranged circumferentially on the body 197a about a rear opening 197h to the body 197a. Each leg 197g comprises an outwardly extending foot 197i.

The nozzle insert body 197a further comprises a second annular section 197j spaced rearwardly of the first annular section 197e and from which the legs 197g depend. The first and second annular sections 197e, 197j are joined together by a plurality of spaced-apart, resilient ribs 197k which are disposed on the outer circumference of the body 197a and extend on a diagonal path between the first and second annular sections 197e, 197j.

The second annular section 197j presents a pair of diametrically opposed, forwardly oriented, resilient tongues 197l. The tongues 197l are disposed between the ribs 197.

On the forward face of the forward end wall 197c there is provided an annular lip 197m about the central aperture 197d. The forward end wall 197c is further provided with apertures 197n therethrough.

The nozzle insert 197 in this embodiment is injection moulded from polypropylene (PP), but could be made from other plastics materials, as will be appreciated by those skilled in the art.

Figures 24B and 24C show the nozzle insert 197 is arranged in the fluid dispenser 110 about the cap 165 so that the sealing tip 160 of the cap 165 projects through the central aperture 197d in the forward end wall 197c of the nozzle insert 197. Moreover, the sealing lip 165h of the cap 165 is slidingly sealingly engaged with the inner circumferential surface of the first annular section 197e of the nozzle insert 197.

The annular space between the nozzle insert 197 and the cap 165 defines the fluid dispensement chamber 146.

It will be seen from Figures 36A-B that the cap 165 is provided with an outwardly projecting, annular flange 165i. As will be appreciated by additional reference to Figures 35A-B and Figure 24B, as the cap 165 is inserted into the nozzle insert 197 during assembly, the flange 165i pushes past the resilient tongues 197l of the nozzle insert 197 to be retained in the space between the first and second annular sections 197e, 197j of the nozzle insert 197.

Mounted on the sealing tip 160 of the cap 165 is the sealing member 154. The sealing member 154 is slidably, sealingly mounted on the sealing tip 160 and seated in the annular lip 197m of the nozzle insert 197. The seal formed between the longitudinal surfaces of the sealing member 154 and the sealing tip 160 is such that fluid cannot pass therebetween.

The sealing member 154 is made from natural rubber or a thermoplastic elastomer (TPE), but other elastic materials may be used which have a 'memory' to return the sealing member 154 to its original state.

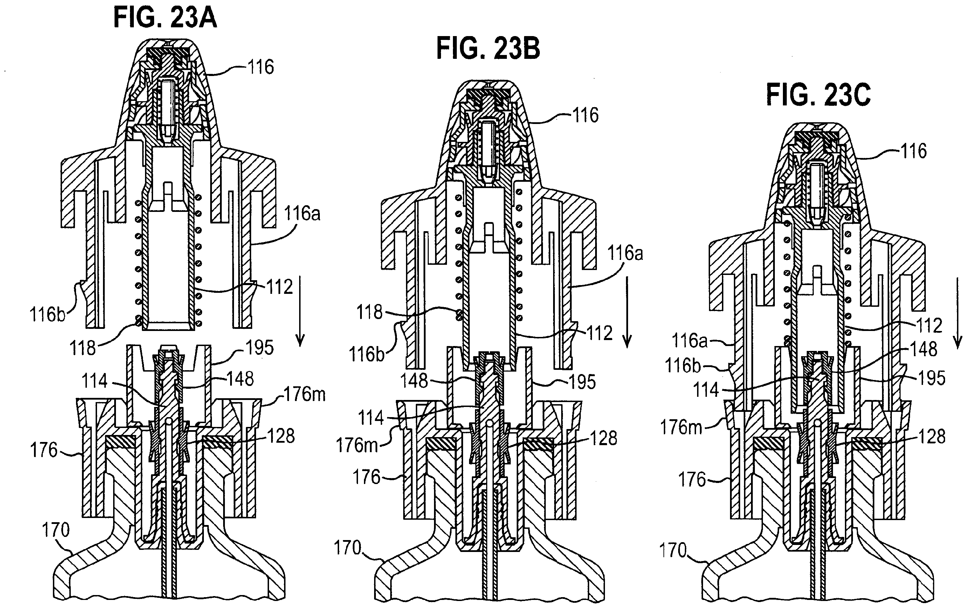

As illustrated by Figures 22 and 23, the nozzle 116 is slidably connected to the stopper portion 176 through engagement of a pair of rearwardly directed runners 116a of the nozzle 116 in complementary tracks 176m on the outer circumference of the stopper portion 176. The runners 116a are provided with outwardly extending clips 116b to secure the runners 116a in the tracks 176m and to delimit the maximum sliding separation between the nozzle 116 and the stopper portion 176.

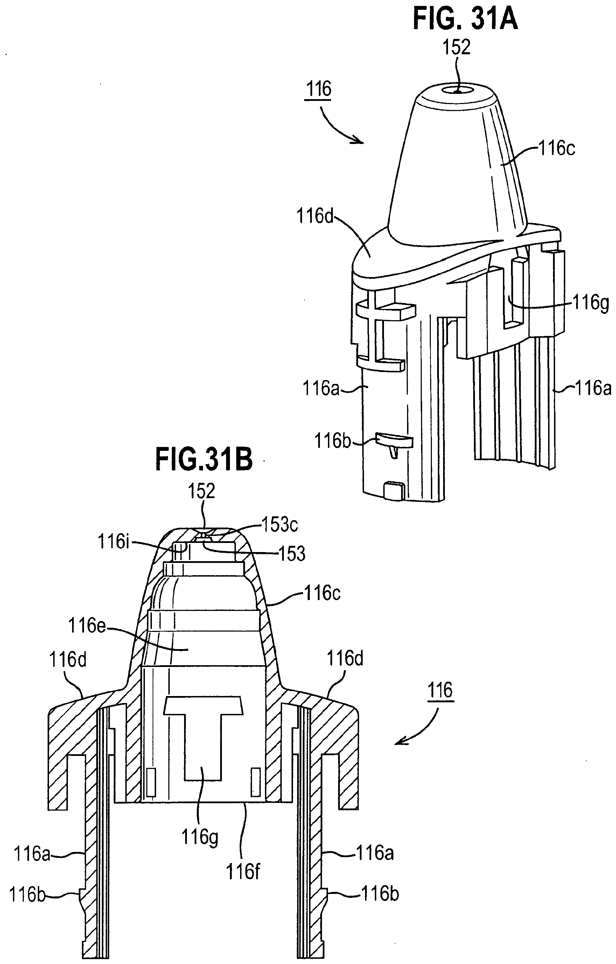

As further illustrated in Figures 31A and 31B, the nozzle 116 has a nozzle section 116c, sized and shaped for insertion into a nostril of a human being, in which is formed the fluid outlet 152, and shoulders 116d at the rear end of the nozzle section 116c from which depend the runners 116a.

The nozzle section 116c encloses an internal cavity 116e having a rear open end 116f. The inner surface of the internal cavity 116e also has a pair of T-shaped tracks 116g on opposite sides of the internal cavity 116e in the longitudinal section of which the clips 195d of the carrier member 195 are clipped to secure the carrier member 195 to the nozzle 116 and to provide for sliding movement therebetween. Moreover, in each corner of the crossbar section of the T-shaped tracks 116g is clipped one of the feet 197i of the nozzle insert 197 to fix the nozzle insert 197 in the internal cavity of the nozzle 116. These connections are best seen in Figures 22A-C.

The resilient ribs 197k of the nozzle insert 197 act as springs to enable the nozzle insert 197 to be inserted into the nozzle 116 and then the second annular section 197j compressed so that the feet 197i fix in the T-shaped tracks 116g. The nozzle insert 197 is then held captive in the nozzle 116. Moreover, the first annular section 197a forms a fluid-tight seal against the adjacent inner surface of the nozzle internal cavity 116e to prevent liquid leaking out of the fluid dispensement chamber 146.

As shown in Figure 32, a swirl chamber 153 is formed in the forward end wall 116i of the nozzle internal cavity 116e. The swirl chamber 153 comprises a central cylindrical chamber 153a and a plurality of feed channels 153b which are equi-spaced about the central chamber 153a in tangential relationship thereto. At the centre of the central chamber 153a is a passageway 153c (exit) connecting the swirl chamber 153 to the fluid outlet 152. The feed channels 153b may have a depth in the range of 100 to 250 microns, for instance in the range of 150 to 225 microns (inclusive).

In Figure 25, there is shown a cross-sectional view of the nozzle area of the fluid dispenser of Figures 22 to 24, but with an alternative tip seal arrangement which, while not used in the claimed invention, is similar to the tip seal arrangement used in the claimed invention and so is useful for understanding its operation.

As will be understood from Figure 25, a gap exists between the side face 154d of the sealing member 154 and the adjacent surfaces of the internal cavity 116e of the nozzle 116 so as to be in flow communication with the fluid dispensement chamber 146 via the apertures 197n and the gaps between the sealing member 154 and the forward opening 197d of the nozzle insert 197.

However, as shown most clearly in Figure 25, the forward face 154c of the flexible sealing member 154 is held by the nozzle insert 197 in sealing engagement with the forward end wall 116i of the nozzle 116. This means that the sealing member 154 seals over the swirl chamber feed channels 153b and that any liquid travelling up the gap between the side face 154d of the sealing member 154 and the nozzle 116 has to pass into the swirl chamber feed channels 153b.

Moreover, the return spring 118 acts to bias the main housing 112 forwardly in the nozzle 116 whereby the sealing tip 160, on the cap 165 fixed on the forward section 112h of the main housing 112, pushes a central part of the forward face 154c of the sealing member 154 into the central chamber 153a of the swirl chamber 153 to sealingly close the passageway 153c to the fluid outlet 152. In this way, no fluid can enter or exit the fluid outlet 152 until the sealing tip 160 releases the central part of the elastic sealing member 154, to be described in more detail hereinafter.

In a modification, the straight walls of the central chamber 153a of the swirl chamber 153 may be chamfered to facilitate pushing the central part of the sealing member 154 thereinto. This is shown in Figure 38, with the chamfered surface denoted by reference number 153d.

The nozzle 116 in this embodiment is injection moulded from polypropylene (PP), but other plastics materials could be used.

To operate the fluid dispenser 110, it is first necessary to prime the device to fill all the fluid pathways between the fluid outlet 152 and the fluid supply 170. To prime, the fluid dispenser 110 is operated in exactly the same manner as for later dispensing operations. As shown in Figures 22B-C and 24B-C, this is done by (i) sliding the nozzle 116 relatively towards the fluid supply 170, by acting on the nozzle 116, or the fluid supply 170, while keeping the other stationary, or acting on both, to move the fluid dispenser from its rest position (Figures 22B and 24B) to its fired position (Figures 22C and 24C); and (ii) allowing the return spring 118 to return the nozzle 116 to its separated position relative to the fluid supply 170 to return the fluid dispenser 110 to its rest position. The relative sliding movement of the nozzle 116 and the fluid supply 170 is effected by the runners 116a of the nozzle 116 sliding in the tracks 176m of the stopper portion 176 fixed in the neck 178 of the fluid supply 170.

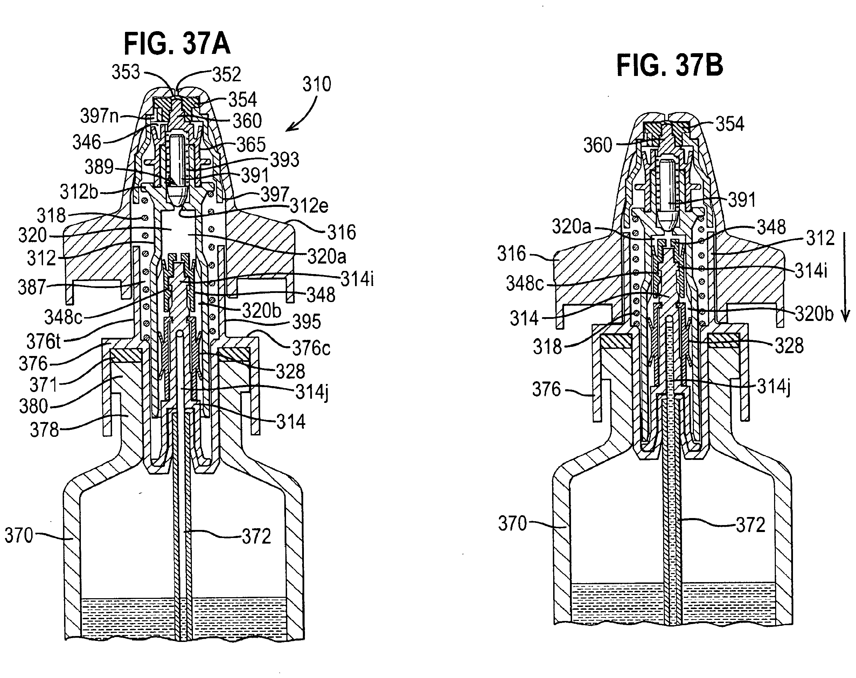

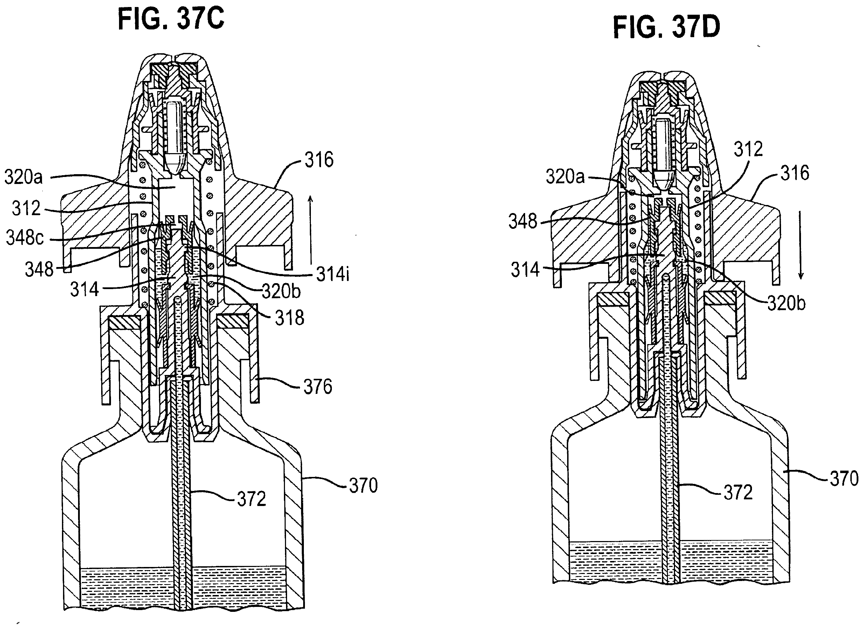

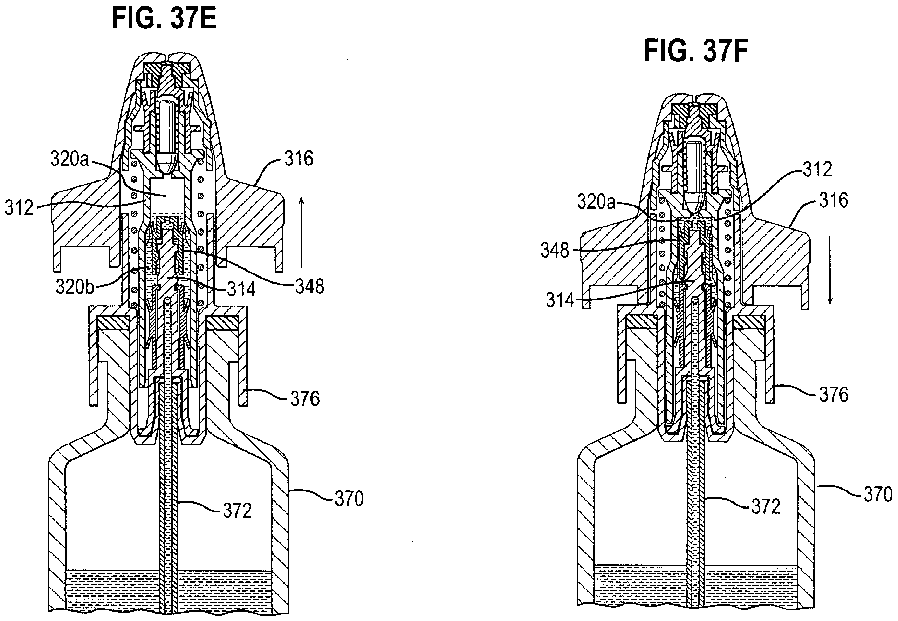

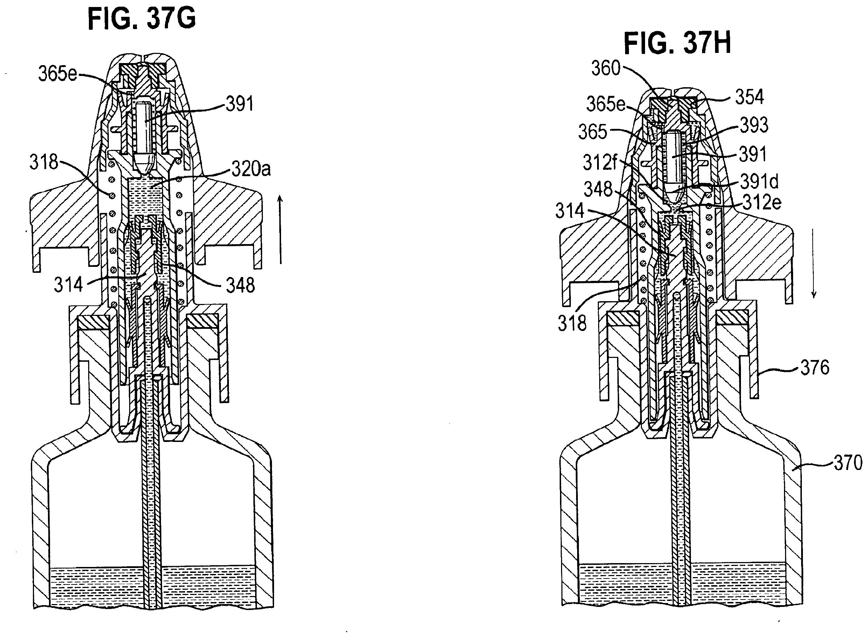

Figures 37A to 37J show the priming process, and the liquid flow during priming, albeit for a fluid dispenser 310 which is a subtle modification (but functional equivalent) of the fluid dispenser 110 of Figures 22 to 36, with like features being assigned like reference numbers. While the fluid dispenser 310 of Figures 37A to 37J will be discussed in more detail after the description of the fluid dispenser 110, Figures 37A to 37J are a useful reference to the detailed description of priming of the fluid dispenser 110 which now follows.

Each complete (reciprocal) cycle of the afore-mentioned sliding movement (a "pumping cycle") between the nozzle 116 and the fluid supply 170 creates a negative pressure in the dosing chamber 120 which draws liquid from the fluid supply 170 up the supply tube 172 until liquid fills up all the fluid pathways from the fluid supply 170 to the fluid outlet 152.

In more detail, the liquid flows forwardly through the supply tube 172, into the bore network 114j of the piston member 114 via the rear opening 114m thereof, and out of the forward openings 114q of the bore network 114j into the rear section 120b of the dosing chamber 120 via the axial grooves 114r in the outer periphery of the piston member 114 (see Figures 37A to 37C).

As a result of the nozzle 116 and the fluid supply 170 respectively carrying the main housing 112 and the piston member 114, as described above, each reciprocal cycle of relative movement of the nozzle 116 and the fluid supply 170 causes the piston member 114 to stroke in corresponding reciprocating fashion inside the dosing chamber 120 defined by the main housing 112 from the rear (rest) position.

As the piston member 114 returns from its forward position to its resting, rear position, in the second half of each cycle, a negative pressure is created in the dosing chamber 120 to draw the liquid further forwardly. Moreover, the piston member 114 moves rearwardly relative to the forward sealing element 148 to open the one-way valve, as described hereinabove, and therefore allows the liquid to flow forwardly into the forward dosing chamber section 120a through the one-way valve (see Figures 37D to 37G).

Specifically, as the annular flange 114i of the piston member 114 disengages from the forward end wall 149d of the central bore section 149c of the bore 149 in the forward sealing element 148, the liquid to the rear of the one-way valve is able to flow around the flange 114i of the piston member 114 via the windows 149f in the forward sealing element 148, over the tip part 114u of the piston member 114 and through the forward bore section 149a of the forward sealing element 148 into the forward section 120a of the dosing chamber 120.

After the dosing chamber 120 (including the forward section 120a) is filled with liquid by priming the fluid dispenser with enough pumping cycles, each cycle thereafter results in the same amount (a metered volume) of the liquid being pumped forward from the dosing chamber 120 through the restricted bore section 112e in the main housing 112.

In more detail, in the forward stroke of the piston member 114 to its forward position in the dosing chamber 120, the valve mechanism 189 in the forward bore section 112f keeps the restricted bore section 112e shut until after the forward sealing element 148 comes into sealing engagement with the inner surface of the forward dosing chamber section 120a. This is because the biasing force of the valve return spring 193 is not overcome by the hydraulic pressure of the liquid produced on the initial (first) phase of the forward stroke of the piston member 114 prior to the forward sealing element 148 sliding into sealing engagement in the forward dosing chamber section 120a to sealingly separate the forward and rear dosing chamber sections 120a, 120b.

This first phase may be referred to as the "bleed phase" because it results in liquid being pumped rearwardly from the dosing chamber 120 back into the fluid supply 170 (i.e. bled) until the piston member 114 locates the forward sealing element 148 in the forward dosing chamber 120a.

Once the forward sealing element 148 is located in the forward dosing chamber 120a, the forward dosing chamber 120a, and the liquid which fills it, is sealed.

In the next (second) phase of the continuous forward stroke of the piston member 114, the piston member 114 increases the hydraulic pressure of the liquid in the forward dosing chamber section 120a as it moves relatively towards the forward end wall 120c of the forward dosing chamber section 120a presented by the annular shoulder 112d of the main housing 112. In other words, the liquid is compressed as the distance between the piston member 114 and the forward end wall 120c of the dosing chamber 120 decreases. Again, this is because the biasing force of the return spring 193 of the valve mechanism 189 resists the increasing hydraulic pressure exerted by the liquid on the frusto-conical rear portion 191d of the valve element 191.

However, at a certain point in the forward stoke of the piston member 114, the hydraulic pressure of the liquid in the forward dosing chamber section 120a is at a level which is greater than the biasing force in the return spring 193 of the valve mechanism 189, whereby the valve element 191 is forced out of sealing engagement with the restricted bore section 112e (which functions as a "valve seat"). This is the start of the final (third) phase of the forward stroke of the piston member 114 which ends when the piston member 114 reaches its forward position, as delimited by abutment of the forward end 148c of the forward sealing element 148 with the forward end wall 120c of the dosing chamber 120. In this final phase, the metered volume of the liquid is dispensed through the restricted bore section 112e, being conveyed along the grooves 191e in the valve member 191 into the forward bore section 112f of the main housing 112, before the valve mechanism 189 is re-closed by the return spring 193 returning the valve member 191 into sealing engagement in the restricted bore section 112e (see Figure 37H).

The valve mechanism 189 only opens in this final (third) phase, remaining closed at all other times.

The second and third phases can collectively be considered as a "dispensing phase".

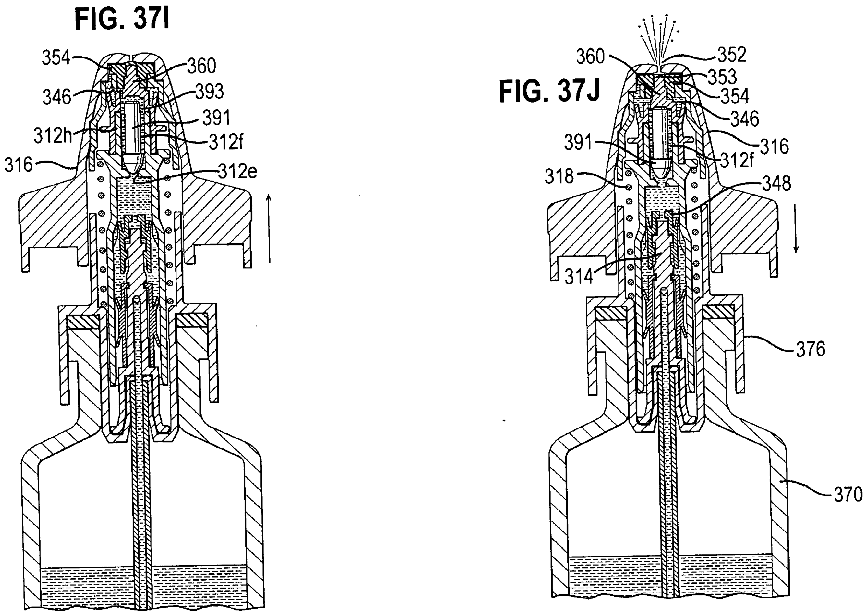

In an initial (first) phase of the return, rearward stroke of the piston member 114 in the dosing chamber 120, the piston member 114 not only moves rearwardly with respect to the dosing chamber 120, but also to the forward sealing element 148 so as to open the one-way valve, as discussed hereinabove. Moreover, a negative pressure (or vacuum) is generated in the headspace being formed in the forward dosing chamber section 120a in front of the rearwardly moving piston member 114. This negative pressure draws more liquid out of the fluid supply 170 and through the open one-way valve into the forward dosing chamber section 120a until the forward sealing element 148 disengages from the forward dosing chamber 120a to enter the rear dosing chamber section 120b (see Figure 37I). The provision of the one-way valve which opens in the initial phase of the return stroke avoids the creation of any hydraulic lock in front of the piston member 114 which could otherwise prevent or inhibit the return stroke.

In a final (second) phase of the rearward stroke of the piston member 114, the piston member 114 moves from an intermediate position, at which the forward sealing element 148 has just been disposed in the rear dosing chamber section 120b, to its rearward position. In this final phase, the liquid is able to be drawn from the rear dosing chamber section 120b directly into the forward dosing chamber section 120a around the outside of the forward sealing element 148, in addition to via the open one-way valve.

At the end of the return, rearward stroke, the dosing chamber 120 is refilled with liquid. The return stroke may thus be referred to as the "filling phase".

In each subsequent cycle of movement of the piston member 114, the forward stroke results in another metered volume of the liquid being discharged through the restricted bore section 112e while the rearward stroke results in another metered volume of liquid being drawn from the fluid supply 170 to refill the forward section 120a of the dosing chamber 120.

During priming, such subsequent pumping cycles continue until the liquid fills the fluid flow path from the dosing chamber 120 to the fluid outlet 152 (see Figure 37I). In this regard, the liquid passing through the restricted bore section 112e flows through the forward bore section 112f of the main housing 112, into the fluid dispensement chamber 146 via the apertures 165e in the forward end wall 165b of the cap 165 mounted over the forward end of the main housing 112, and then into the space around the sealing member 154 by passing through the apertures 197n in the nozzle insert 197 fitted inside the nozzle 116 to enclose the cap 165.

When liquid fills the fluid pathway from the fluid supply 170 to the fluid outlet 152, the forward stroke of the piston member 114 relative to the dosing chamber 120 in the next pumping cycle results in another metered volume of liquid being pumped through the restricted bore section 112e thereby pressurising the liquid pending downstream of the restricted bore section 112e. This pressure in the fluid dispensement chamber 146 results in rearward sliding movement of the cap 165 (and the main housing 112) in the nozzle insert 197 against the return force of the return spring 118 whereby the sealing tip 160 sealingly slides rearwardly in the sealing member 154. As a result, the elasticity of the sealing member 154 flattens the central part of the forward face 154c of the sealing member 154 back to its original state to open the central chamber 153a and passageway 153c of the swirl chamber 153. Consequently, a metered volume of the liquid is pumped through the fluid outlet 152 via the swirl chamber 153 for atomisation thereof to make space for the metered volume pumped through the restricted bore section 112e in that forward stroke (see Figure 37J).

The seal between the opposing longitudinal sides of the sealing tip 160 and the sealing member 154 prevents liquid under the hydraulic pressure entering the sealing member cavity 154e (Figure 25) in which the sealing tip 160 is disposed and acting to oppose the central part of the forward face 154c of the sealing member 154 moving back to its original state when released by the sealing tip 160.

The return force of the return spring 118 moves the main housing 112 back (forwardly) to its normal, rest position in the nozzle insert 197 once the return force is greater than the hydraulic pressure in the fluid dispensement chamber 146 so that the sealing tip 160 deflects the sealing member 154 to (re)close the fluid outlet 152.

The sealing member 154 thus protects the liquid inside the fluid dispenser 110 from contamination by contaminants outside of the device 110 entering through the fluid outlet 152 as it only opens during dispensing (i.e. when the fluid dispenser 110 is fired).

The rearward stroke of the same pumping cycle draws another metered volume of liquid from the liquid supply 170 to fill the dosing chamber 120, ready for the next pump cycle.

The device is now fully primed, and each pump cycle thereafter results in a constant metered volume of the liquid being pumped from the fluid outlet 152 until the fluid supply 170 is exhausted.

It will be appreciated that the fluid dispenser 110 configuration is such that there will be no drain-back of the liquid pending in the path between the dosing chamber 120 and the fluid outlet 152 as the restricted bore section 112e is sealed shut by the valve mechanism 189 except in the dispensing phase of the forward stroke. Thus, the need to re-prime the device is avoided or substantially alleviated. Moreover, the tip seal arrangement, formed by the sealing member 154 and the sealing tip 160, and the valve mechanism 189 prevent ambient air being drawn into the fluid dispenser 110 through the fluid outlet 152 by the negative pressure (e.g. vacuum) created in the dosing chamber 120 in the filling phase.

It is also notable that during priming of the fluid dispenser 110, air (and any other gas) in the headspace above the liquid is pumped out of the fluid outlet 152 by the same mechanism as described above for the liquid.

Figures 22A and 24A show the fluid dispenser 110 in an open (fully extended) position, where the nozzle 116 (and its attached components) is spaced farther from the bottle 170 (and its attached components) than in the rest position shown in Figures 22B and 24B. More particularly, in the rest position, the carrier member 195 rests on, or in close proximity to, the roof 176c of the stopper portion 176, whereas in the open position the carrier member 195 is spaced from the stopper portion roof 176c. In the open position, the clips 116b on the runners 116a of the nozzle 116 are at the forwardmost position with respect to the tracks 176m on the stopper portion 176, as shown in Figure 24A. In the rest position, by contrast, the clips 116b are spaced rearwardly of the forwardmost position, as also shown in Figure 24B. The ability for the nozzle 116 and bottle 170 to be further separated from the normal rest position provides protection of the fluid dispenser against breakage in the event it is dropped or suffers an impact.

There now follows descriptions of alternative sealing arrangements that could be used in the fluid dispenser 110, with like reference numerals being used to indicate like parts and features with the sealing arrangement in Figures 22 to 36.

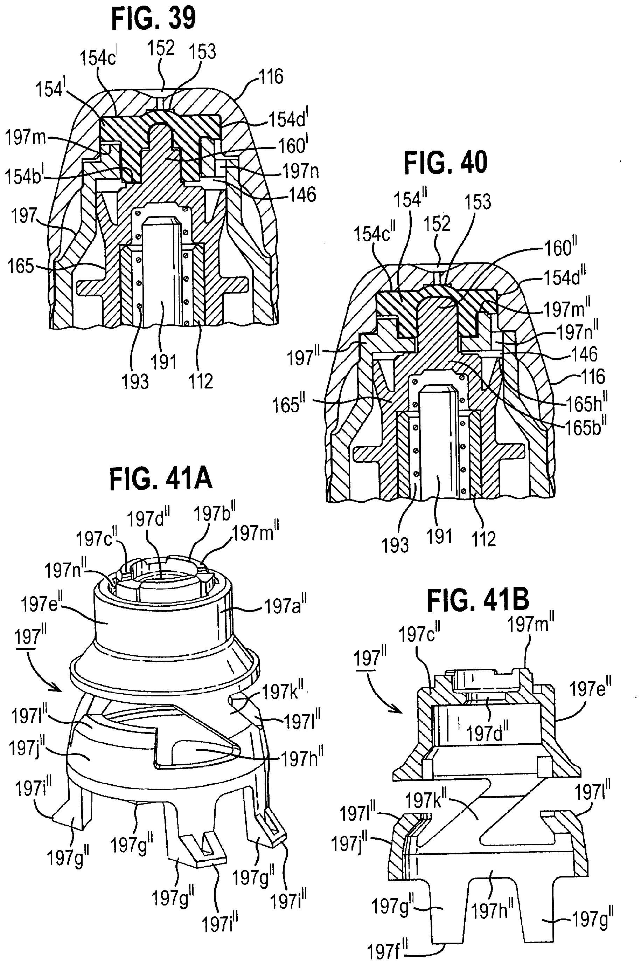

In Figure 39 there is shown a first alternative tip seal arrangement that could be used in the fluid dispenser 110. In Figure 39, the sealing tip 160' and sealing member 154' are of different shape compared to their counterparts in the fluid dispenser 110 of Figures 22 to 36, but function in the same way as their counterparts. The cap 160 and sealing member 154' are of the same materials as described for the fluid dispenser 110 of Figures 22 to 36.

In Figures 40 and 41A-B there is shown a second alternative tip seal arrangement that could be used in the fluid dispenser 110. In this alternative, the cap 165", sealing member 154" and nozzle insert 197" are of different shape to their counterparts in the fluid dispenser 110 of Figures 22 to 36, but again function in the same way, and are made from the same materials, as those counterparts. However, in this version of the tip seal arrangement, the return spring 118 biases the cap 165" into abutment with the nozzle insert 197" to control the position of the sealing tip 160" relative to the sealing member 154", thereby protecting the sealing member 154" from excessive force being applied to it by the sealing tip 160".

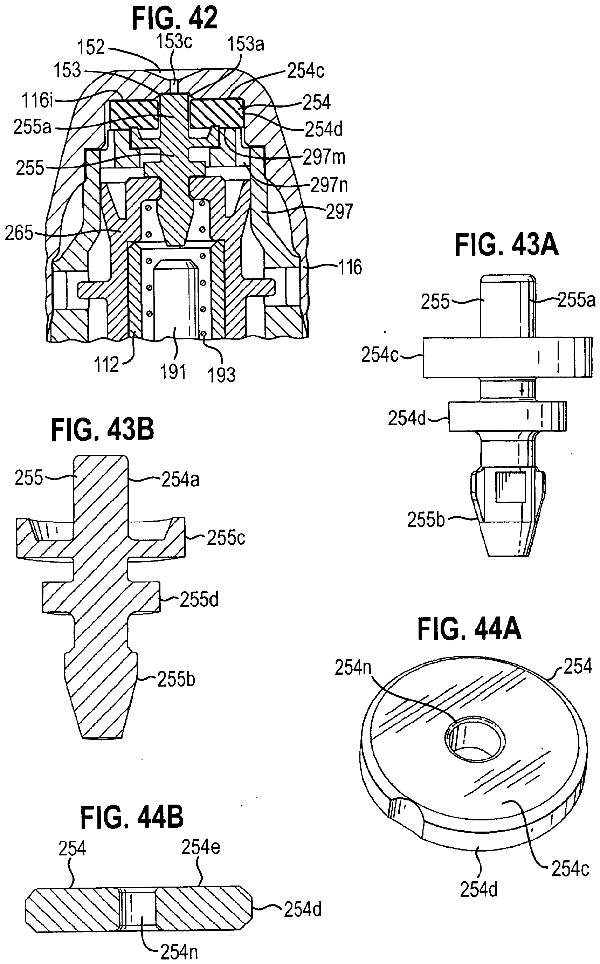

In Figure 42 there is shown a different type of sealing arrangement for the fluid dispenser 110, with Figures 43 to 46 showing the components for this sealing arrangement.

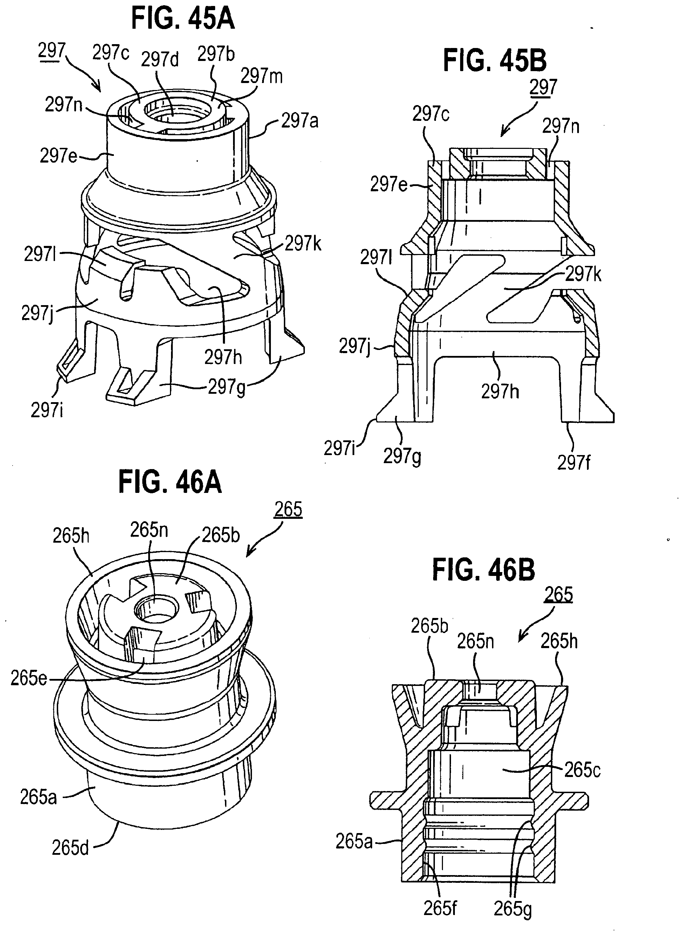

In place of the elastic sealing member 154 there is provided an annular backing plate 254 (Figures 44A-B), made from a plastics material. In this embodiment, the backing plate is injection moulded from polypropylene (PP). The forward face 254c of the backing plate 254 is held by a modified nozzle insert 297 (Figures 45A-B) in sealing engagement with the forward end wall 116i of the nozzle 116 so as to seal over the swirl chamber feed channels 153b whereby any liquid travelling up the gap between the side face 254d of the backing plate 254 and the nozzle 116 has to pass into the swirl chamber feed channels 153b.

A sealing pin 255 (Figures 43A-B) is seated on the nozzle insert 297 so that a forward sealing section 255a of the sealing pin 255 protrudes through the through-hole 254n in the backing plate 254 and into the central chamber 153a of the swirl chamber 153 to sealing close the passageway 153c. Thus, the sealing pin 255 functions similarly to the elastic sealing member 154.

As shown in Figure 42, the sealing pin 255 has an enlarged, rear end 255b of tapering profile which is held captive in a through-hole 265n in the forward end wall 265b of a modified cap 265 (Figures 46A-B) so that the sealing pin 255 moves in unison with the main housing 112 to which the cap 265 is fixed.

It will therefore be appreciated that the return spring 118 acts on the main housing 112 to bias the sealing pin 255 into sealing engagement over the swirl chamber passageway 153c. Moreover, during the dispensing phase of the forward stroke of the piston member 114 in the dosing chamber 120, the hydraulic pressure produced in the fluid dispensement chamber 146 results in the cap 265 moving rearwardly against the return spring force, and in do doing moves the sealing pin 255 rearwardly so as to open the swirl chamber passageway 153c for release of the metered volume of liquid.

It will be observed that the sealing pin 255 is provided with forward and rear annular flanges 255c, 255d. The rear flange 255d delimits the insertion of the sealing pin 255 into the cap through-hole 265n. The forward flange 255c seals against the rear side of the backing plate 254.

It will further be observed that the valve element 191 of the valve mechanism 189 in the main housing 112 is provided with an abbreviated length to accommodate the sealing pin 255.

The sealing pin 255 in this embodiment is injection moulded from low density polyethylene (LDPE) or high density polyethylene (HDPE), but other functionally equivalent plastics materials could be used.

The modified cap 265 and modified nozzle insert 297 are made from the same materials are described for the corresponding parts in the fluid dispenser 110 of Figures 22 to 36. The modified nozzle insert 297 may also have a castellated forward end wall 297c, as in the other illustrated nozzle inserts 197; 197'; 197'.

Referring now to the fluid dispenser 310 shown in Figures 37A-J, this functions in the same way as the fluid dispenser 110 of Figures 22 to 36. The sealing tip 360, sealing member 354, forward sealing element 328 and stopper portion 376 are of a slightly different structure to the corresponding components in the fluid dispenser 110. Most notably, however, is the absence of a carrier member for the return spring 318 in the fluid dispenser 310. It will be seen from Figure 37A that an annular retaining wall 376t projects forwardly from the roof 376c of the stopper portion 376. As further shown in Figure 37A, the return spring 318 is carried on the stopper portion roof 376c and extends forwardly to the annular flange 312b of the main housing 312 through the gap formed between the annular retaining wall 376t and the main housing 312. It will therefore be appreciated that the fluid dispenser 310 does not have an open position, like the fluid dispenser 110, for improving protection against damage if dropped or otherwise impacted.

In the exemplary embodiments the sealing arrangement at the fluid outlet 52; 152; etc of the fluid dispenser 10; 110; etc acts to prevent or inhibit the ingress of microbials and other contaminants into the dispenser 10; 110; etc through the fluid outlet 52; 152; etc, and hence into the dosing chamber 20; 120; etc and ultimately the bottle/reservoir of the fluid. Where the fluid is a liquid medicament formulation, e.g. for nasal administration, this enables the formulation to be free of preservatives or, perhaps more likely, to be a preservative-sparing formulation. In addition, the seal acts to prevent the pending dose of the fluid in the dosing chamber from draining back into the supply or reservoir when the dispenser 10; 110; etc is in its rest configuration between actuations. This avoids or reduces the need for the dispenser 10 to be primed for its next usage (priming then only effectively being required for the very first usage of the fluid dispenser so as to fill the dosing chamber 20; 120; etc, but not after the first usage).

The fluid dispenser of the invention may be used to dispense a liquid medicament formulation for the treatment of mild, moderate or severe acute or chronic symptoms or for prophylactic treatment. The precise dose administered will depend on the age and condition of the patient, the particular medicament used and the frequency of administration and will ultimately be at the discretion of the attendant physician. When combinations of medicaments are employed the dose of each component of the combination will in general be that employed for each component when used alone.

Appropriate medicaments for the formulation may be selected from, for example, analgesics, e.g., codeine, dihydromorphine, ergotamine, fentanyl or morphine; anginal preparations, e.g., diltiazem; antiallergics, e.g., cromoglycate (eg as the sodium salt), ketotifen or nedocromil (eg as the sodium salt); antiinfectives e.g., cephalosporins, penicillins, streptomycin, sulphonamides, tetracyclines and pentamidine; antihistamines, e.g., methapyrilene; anti- inflammatories, e.g., beclomethasone (eg as the dipropionate ester), fluticasone (eg as the propionate ester), flunisolide, budesonide, rofleponide, mometasone (eg as the furoate ester), ciclesonide, triamcinolone (eg as the acetonide), 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester or 6α, 9α-Difluoro-17α-[(2-furanylcarbonyl)oxy]-11β-hydroxy-16α-methyl-3-oxo-androsta-1,4-diene-17β-carbothioic acid S-fluoromethyl ester; antitussives, e.g., noscapine; bronchodilators, e.g., albuterol (eg as free base or sulphate), salmeterol (eg as xinafoate), ephedrine, adrenaline, fenoterol (eg as hydrobromide), formoterol (eg as fumarate), isoprenaline, metaproterenol, phenylephrine, phenylpropanolamine, pirbuterol (eg as acetate), reproterol (eg as hydrochloride), rimiterol, terbutaline (eg as sulphate), isoetharine, tulobuterol or 4-hydroxy-7-[2-[[2-[[3-(2-phenylethoxy)propyl]sulfonyl]ethyl]amino]ethyl-2(3H)-benzothiazolone; PDE4 inhibitors eg cilomilast or roflumilast; leukotriene antagonists eg montelukast, pranlukast and zafirlukast; [adenosine 2a agonists, eg 2R,3R,4S,5R)-2-[6-Amino-2-(1S-hydroxymethyl-2-phenyl-ethylamino)-purin-9-yl]-5-(2-ethyl-2H-tetrazol-5-yl)-tetrahydro-furan-3,4-diol (e.g. as maleate)]*; [α4 integrin inhibitors eg (2S)-3-[4-({[4-(aminocarbonyl)-1-piperidinyl]carbonyl}oxy)phenyl]-2-[((2S)-4-methyl-2-{[2-(2-methylphenoxy) acetyl]amino}pentanoyl)amino] propanoic acid (e.g as free acid or potassium salt)]*, diuretics, e.g., amiloride; anticholinergics, e.g., ipratropium (eg as bromide), tiotropium, atropine or oxitropium; hormones, e.g., cortisone, hydrocortisone or prednisolone; xanthines, e.g., aminophylline, choline theophyllinate, lysine theophyllinate or theophylline; therapeutic proteins and peptides, e.g., insulin or glucagons. It will be clear to a person skilled in the art that, where appropriate, the medicaments may be used in the form of salts, (e.g., as alkali metal or amine salts or as acid addition salts) or as esters (e.g., lower alkyl esters) or as solvates (e.g., hydrates) to optimise the activity and/or stability of the medicament and/or to minimise the solubility of the medicament in the propellant.

Preferably, the medicament is an anti-inflammatory compound for the treatment of inflammatory disorders or diseases such as asthma and rhinitis.

In one aspect, the medicament is a glucocorticoid compound, which has anti-inflammatory properties. One suitable glucocorticoid compound has the chemical name: 6α, 9α-Difluoro-17α-(1-oxopropoxy)-11β-hydroxy-16α-methyl-3-oxo-androsta-1,4-diene-17β-carbothioic acid *S*-fluoromethyl ester (fluticasone propionate). Another suitable glucocorticoid compound has the chemical name: 6α, 9α-difluoro-17α-[(2-furanylcarbonyl)oxy]-11β-hydroxy-16α-methyl-3-oxo-androsta-1,4-diene-17β-carbothioic acid *S*-fluoromethyl ester. A further suitable glucocorticoid compound has the chemical name: 6α,9β-Difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester.

Other suitable anti-inflammatory compounds include NSAIDs e.g. PDE4 inhibitors, leukotriene antagonists, iNOS inhibitors, tryptase and elastase inhibitors, beta-2 integrin antagonists and adenosine 2a agonists.

Other medicaments which may be comprised in the formulation are 6-({3-[(Dimethylamino)carbonyl]phenyl}sulfonyl)-8-methyl-4-{[3-(methyloxy) phenyl]amino}-3-quinolinecarboxamide; 6a,9a-Difluoro-11b-hydroxy-16a-methyl-17a-(1-methycyclopropylcarbonyl)oxy-3-oxo-androsta-1,4-diene-17b-carbothioic acid *S*-fluoromethyl ester; 6a,9a-Difluoro-11i-hydroxy-16a-methyl-3-oxo-17a-(2,2,3,3- tetramethycyclopropylcarbonyl)oxy-androsta-1,4-diene-17i-carbothioic acid *S*-cyanomethyl ester; 1-{[3-(4-{[4-[5-fluoro-2-(methyloxy)phenyl]-2-hydroxy-4-methyl-2-(trifluoromethyl)pentyl] amino -6-methyl-1H-indazol-1-yl)phenyl]carbonyl}-D-prolinamide; and the compound disclosed in International patent application No. PCT/EP2007/053773, filed 18th April 2007, in Example 24, and in particular the form which is 24C therein.

The fluid dispenser herein is suitable for dispensing fluid medicament formulations for the treatment of inflammatory and/or allergic conditions of the nasal passages such as rhinitis e.g. seasonal and perennial rhinitis as well as other local inflammatory conditions such as asthma, COPD and dermatitis.

A suitable dosing regime would be for the patient to inhale slowly through the nose subsequent to the nasal cavity being cleared. During inhalation the formulation would be applied to one nostril while the other is manually compressed. This procedure would then be repeated for the other nostril. Typically, one or two inhalations per nostril would be administered by the above procedure up to three times each day, ideally once daily. Each dose, for example, may deliver 5µg, 50µg, 100µg, 200µg or 250µg of active medicament. The precise dosage is either known or readily ascertainable by those skilled in the art.

All usage herein of terms such as "about", "approximately", "substantially" and the like in relation to a parameter or property is meant to include the exact parameter or property as well as immaterial deviations therefrom.

The embodiments of the present invention described above are purely illustrative. Modifications in detail may be made within the scope of the invention as defined in the claims and the Summary of the Invention.

## Claims

1. A fluid dispenser (110) having a fluid outlet (152) through which fluid will, in use, be dispensed, a seal member (154") for sealing the fluid outlet which is movable from a normal closed state, in which the seal member prevents fluid being dispensed through the fluid outlet, to an open state, in which the seal member opens the fluid outlet for dispensing therefrom, and a member (165") movable between a normal first position, in which the member acts on the seal member to locate the seal member in the closed state, and a second position, which enables the seal member to move to the open state, **characterised by** the dispenser having a biasing mechanism (118) which biases the member to its first position, and further in that the dispenser is adapted such that the member moves from the first position to the second position in response to actuation of the fluid dispenser, that the seal member is supported on a support member (197") having a passageway (197d") therethrough, the member moves between the first and second positions relative to the support member, that the member has a free end (160") which, in the first position, extends into the passageway to push against the seal member to push the seal member into sealing engagement with the fluid outlet, and that the member and the support member are interengageable to limit how far the free end is able to extend into the passageway to push against the seal member.

2. The fluid dispenser of claim 1, wherein the member is a piston member.

3. The fluid dispenser of claim 1, wherein the seal member is resilient having a normal state, the closed state is an abnormal state of the seal member and the seal member moves back towards its normal state to move from the closed state to the open state.

4. The fluid dispenser of claim 3, wherein the member holds the seal member in its abnormal state when in its first position.

5. The fluid dispenser of any of claims 1 to 4, adapted on actuation to pressurise fluid in the dispenser to cause movement of the seal member from its closed state to its open state.

6. The fluid dispenser of claim 5, adapted such that the pressurised fluid acts to move the member from its first position to its second position.

7. The fluid dispenser of any preceding claim, wherein the free end comprises a nipple (160") which acts on the seal member to locate the seal member in the closed state.

8. The fluid dispenser of any preceding claim, wherein the member sealingly slides on the support member as it moves between its first and second positions, wherein a chamber (146) is defined between the member and the support member which forms part of the fluid flow path to the fluid outlet and in use contains fluid, and wherein the dispenser is adapted on actuation to pressurise fluid present in the chamber to drive the member to its second position.

9. The fluid dispenser of claim 8, wherein the support member provides at least one fluid passageway (197n") for fluid to flow from the chamber towards the fluid outlet upon movement of the member from the first position to the second position.

10. The fluid dispenser of any preceding claim, wherein the fluid outlet comprises a swirl chamber (153) having a central chamber (153a), an exit passageway (153c) from the central chamber and at least one feed channel (153b) for the central chamber, wherein the seal member is supported on the support member such as to lie against the swirl chamber and wherein the member pushes a central part of the seal member into the central chamber of the swirl chamber.

11. The fluid dispenser of any preceding claim, wherein the support member has a wall and wherein the member has a shoulder which is interengageable with the support member wall to limit the amount to which the free end extends into the passageway.

12. The fluid dispenser of claim 11, wherein the passageway is formed in the wall.

13. The fluid dispenser of any preceding claim, wherein the support member has a hollow body comprising an end wall through which the passageway is provided, wherein the seal member is seated on an outer side of the end wall and wherein the member is interengageable with an inner side of the end wall to limit how far the free end is able to extend into the passageway to push against the seal member.

14. The fluid dispenser of any preceding claim, wherein the free end of the member extends into the passageway so as to protrude therefrom to push against the seal member.

15. The fluid dispenser of any preceding claim, wherein in the first position of the member, the member and the support member are interengaged to limit how far the free end extends into the passageway to push against the seal member.

## Patentansprüche

1. Fluidspender (110) mit einem Fluidauslass (152), durch welchen bei Verwendung ein Fluid abgegeben wird, einem Dichtelement (154") zum Abdichten des Fluidauslasses, welches von einem normalen geschlossenen Zustand, in welchem das Dichtelement verhindert, dass Fluid durch den Fluidauslass abgegeben wird, zu einem offenen Zustand bewegbar ist, in welchem das Dichtelement den Fluidauslass zur Abgabe davon öffnet, und einem Element (165"), das zwischen einer normalen ersten Position, in welcher das Element auf das Dichtelement wirkt, um das Dichtelement in dem geschlossenen Zustand festzulegen, und einer zweiten Position bewegbar ist, welche dem Dichtelement ermöglicht, sich zu dem offenen Zustand zu bewegen, **dadurch gekennzeichnet, dass** der Spender einen Vorspannmechanismus (118) aufweist, welcher das Element zu seiner ersten Position vorspannt, und ferner dadurch, dass der Spender derart angepasst ist, dass sich das Element als Reaktion auf eine Betätigung des Fluidspenders von der ersten Position zu der zweiten Position bewegt, dass das Dichtelement an einem Stützelement (197") mit einem Durchgang (197d") dadurch gestützt ist, sich das Element zwischen der ersten und der zweiten Position relativ zu dem Stützelement bewegt, dass das Element ein freies Ende (160") aufweist, welches sich in der ersten Position in den Durchgang erstreckt, um gegen das Dichtelement zu drücken, um das Dichtelement in einen dichtenden Eingriff mit dem Fluidauslass zu drücken, und dass das Element und das Stützelement miteinander in Eingriff bringbar sind, um zu begrenzen wie weit sich das freie Ende in den Durchgang erstrecken kann, um gegen das Dichtelement zu drücken.

2. Fluidspender nach Anspruch 1, wobei das Element ein Kolbenelement ist.

3. Fluidspender nach Anspruch 1 oder 2, wobei das Dichtelement elastisch mit einem normalen Zustand ist, wobei der geschlossene Zustand ein anormaler Zustand des Dichtelements ist und sich das Dichtelement zurück zu seinem normalen Zustand hin bewegt, um sich von dem geschlossenen Zustand zu dem offenen Zustand zu bewegen.

4. Fluidspender nach Anspruch 3, wobei das Element das Dichtelement in seinem anormalen Zustand hält, wenn es in seiner ersten Position ist.

5. Fluidspender nach einem der Ansprüche 1 bis 4, angepasst, bei Betätigung ein Fluid in dem Spender unter Druck zu setzen, um eine Bewegung des Dichtelements von seinem geschlossenen Zustand zu seinem offenen Zustand zu bewirken.

6. Fluidspender nach Anspruch 5, derart angepasst, dass das unter Druck gesetzte Fluid wirkt, um das Element von seiner ersten Position zu seiner zweiten Position zu bewegen.

7. Fluidspender nach einem der vorhergehenden Ansprüche, wobei das freie Ende einen Nippel (160") umfasst, welcher auf das Dichtelement wirkt, um das Dichtelement in dem geschlossenen Zustand festzulegen.

8. Fluidspender nach einem der vorhergehenden Ansprüche, wobei das Element an dem Stützelement abdichtend gleitet, wenn es sich zwischen seinen ersten und zweiten Positionen bewegt, wobei eine Kammer (146) zwischen dem Element und dem Stützelement definiert ist, welche Teil des Fluidströmungspfads zu dem Fluidauslass bildet und bei Verwendung ein Fluid enthält, und wobei der Spender angepasst ist, bei Betätigung in der Kammer vorhandenes Fluid unter Druck zu setzen, um das Element zu seiner zweiten Position anzutreiben.

9. Fluidspender nach Anspruch 8, wobei das Stützelement zumindest einen Fluiddurchgang (197n") vorsieht, für Fluid, um bei Bewegung des Elements von der ersten Position zu der zweiten Position von der Kammer zu dem Fluidauslass hin zu strömen.

10. Fluidspender nach einem der vorhergehenden Ansprüche, wobei der Fluidauslass eine Wirbelkammer (153) umfasst, die eine zentrale Kammer (153a), einen Ausgangsdurchgang (153c) von der zentralen Kammer und zumindest einen Zuführkanal (153b) für die zentrale Kammer aufweist, wobei das Dichtelement an dem Stützelement gestützt ist, um gegen die Wirbelkammer zu liegen, und wobei das Element einen zentralen Teil des Dichtelements in die zentrale Kammer der Wirbelkammer drückt.

11. Fluidspender nach einem der vorhergehenden Ansprüche, wobei das Stützelement eine Wand aufweist, und wobei das Element eine Schulter aufweist, welche mit der Stützelementwand miteinander in Eingriff bringbar ist, um den Betrag zu begrenzen, um welchen sich das freie Ende in den Durchgang erstreckt.

12. Fluidspender nach Anspruch 11, wobei der Durchgang in der Wand ausgebildet ist.

13. Fluidspender nach einem der vorhergehenden Ansprüche, wobei das Stützelement einen hohlen Körper mit einer Endwand aufweist, durch welche der Durchgang vorgesehen ist, wobei das Dichtelement an einer Außenseite der Endwand sitzt, und wobei das Element mit einer Innenseite der Endwand miteinander in Eingriff bringbar ist, um zu begrenzen, wie weit sich das freie Ende in den Durchgang erstrecken kann, um gegen das Dichtelement zu drücken.

14. Fluidspender nach einem der vorhergehenden Ansprüche, wobei sich das freie Ende des Elements in den Durchgang erstreckt, um davon vorzustehen, um gegen das Dichtelement zu drücken.

15. Fluidspender nach einem der vorhergehenden Ansprüche, wobei das Element und das Stützelement in der ersten Position des Elements miteinander im Eingriff stehen, um zu begrenzen, wie weit sich das freie Ende in den Durchgang erstreckt, um gegen das Dichtelement zu drücken.

## Revendications

1. Distributeur de fluide (110) ayant un refoulement de fluide (152) à travers lequel un fluide sera, en utilisation, distribué, un organe formant joint (154") pour étanchéifier le refoulement de fluide qui est mobile d'un état fermé normal, dans lequel l'organe formant joint empêche le fluide d'être distribué à travers le refoulement de fluide, à un état ouvert, dans lequel l'organe formant joint ouvre le refoulement de fluide pour dispense depuis celui-ci, un organe (165") mobile entre une première position normale, dans laquelle l'organe agit sur l'organe formant joint pour placer l'organe formant joint dans l'état fermé, et une seconde position, qui permet à l'organe formant joint de se déplacer vers l'état ouvert, **caractérisé en ce que** le distributeur comporte un mécanisme de sollicitation (118) qui sollicite l'organe vers sa première position, et en outre **en ce que** le distributeur est adapté pour que l'organe se déplace de la première position à la seconde position en réponse à un actionnement du distributeur de fluide, **en ce que** l'organe formant joint est supporté sur un organe de support (197") traversé par un passage (197d"), l'organe se déplace entre les première et seconde positions par rapport à l'organe de support, **en ce que** l'organe comporte une extrémité libre (160") qui, dans la première position, s'étend dans le passage pour pousser l'organe formant joint en vue de pousser l'organe formant joint en engagement d'étanchéité avec le refoulement de fluide, et **en ce que** l'organe et l'organe de support peuvent s'engager mutuellement pour limiter l'ampleur à laquelle l'extrémité libre est apte à s'étendre dans le passage pour pousser contre l'organe formant joint.

2. Distributeur de fluide selon la revendication 1, dans lequel l'organe est un organe formant piston.

3. Distributeur de fluide selon la revendication 1 ou 2, dans lequel l'organe formant joint est résilient ayant un état normal, l'état fermé est un état anormal de l'organe formant joint et l'organe formant joint retourne vers son état normal pour se déplacer de l'état fermé à l'état ouvert.

4. Distributeur de fluide selon la revendication 3, dans lequel l'organe tient l'organe formant joint dans son état anormal lorsqu'il est dans sa première position.

5. Distributeur de fluide selon l'une quelconque des revendications 1 à 4, adapté pour un actionnement en vue de pressuriser un fluide dans le distributeur pour provoquer un mouvement de l'organe formant joint de son état fermé à son état ouvert.

6. Distributeur de fluide selon la revendication 5, adapté pour que le fluide pressurisé agisse pour déplacer l'organe de sa première position à sa seconde position.

7. Distributeur de fluide une quelconque revendication précédente, dans lequel l'extrémité libre comprend un mamelon (160") qui agit sur l'organe formant joint pour placer l'organe formant joint dans l'état fermé.

8. Distributeur de fluide selon une quelconque revendication précédente, dans lequel l'organe glisse avec étanchéité sur l'organe de support à mesure qu'il se déplace entre ses première et seconde positions, dans lequel une chambre (146) est définie entre l'organe et l'organe de support qui fait partie du chemin d'écoulement de fluide vers le refoulement de fluide et en utilisation contient du fluide, et dans lequel le distributeur est adapté pour un actionnement en vue de pressuriser un fluide présent dans la chambre pour entraîner l'organe vers sa seconde position.

9. Distributeur de fluide selon la revendication 8, dans lequel l'organe de support fournit au moins un passage de fluide (197n") pour qu'un fluide s'écoule de la chambre vers le refoulement de fluide lors d'un mouvement de l'organe de la première position à la seconde position.

10. Distributeur de fluide selon une quelconque revendication précédente, dans lequel le refoulement de fluide comprend une chambre de tourbillonnement (153) ayant une chambre centrale (153a), un passage de sortie (153c) depuis la chambre centrale et au moins un canal d'alimentation (153b) pour la chambre centrale, dans lequel l'organe formant joint est supporté sur l'organe de support de manière à reposer contre la chambre de tourbillonnement et dans lequel l'organe pousse une partie centrale de l'organe formant joint dans la chambre centrale de la chambre de tourbillonnement.

11. Distributeur de fluide selon une quelconque revendication précédente, dans lequel l'organe de support comporte une paroi et dans lequel l'organe comporte un épaulement qui peut être engagé mutuellement avec la paroi de l'organe de support pour limiter la portée sur laquelle l'extrémité libre s'étend dans le passage.

12. Distributeur de fluide selon la revendication 11, dans lequel le passage est formé dans la paroi.

13. Distributeur de fluide selon une quelconque revendication précédente, dans lequel l'organe de support comporte un corps creux comprenant une paroi d'extrémité dans laquelle est ménagé le passage, dans lequel l'organe formant joint est assis sur un côté externe de la paroi d'extrémité et dans lequel l'organe peut être engagé mutuellement avec un côté interne de la paroi d'extrémité pour limiter l'ampleur à laquelle l'extrémité libre est apte à s'étendre dans le passage pour pousser contre l'organe formant joint.

14. Distributeur de fluide selon une quelconque revendication précédente, dans lequel l'extrémité libre de l'organe s'étend dans le passage de manière à en dépasser pour pousser contre l'organe formant joint.

15. Distributeur de fluide selon une quelconque revendication précédente, dans lequel dans la première position de l'organe, l'organe et l'organe de support sont engagés mutuellement pour limiter l'ampleur à laquelle l'extrémité libre s'étend dans le passage pour pousser contre l'organe formant joint.
